# EUROPEAN PATENT SPECIFICATION

(11) **EP 1 562 941 B1**
(45) Date of publication and mention of the grant of the patent: **23.12.2009**
(21) Application number: 03768730.8
(22) Date of filing: 07.11.2003
(51) Int. Cl.: C07D 417/06, C07D 413/06, C07D 405/06, A61K 31/427

(54) **TRANS-9,10-DEHYDROEPOTHILONE C AND D, ANALOGS THEREOF AND METHOS OF MAKING THE SAME**
TRANS-9,10-DEHYDROEPOTHILON C UND D, ANALOGA DAVON UND VERFAHREN ZU DEREN HERSTELLUNG
TRANS-9,10-DEHYDROEPOTHILONES C ET D, LEURS ANALOGUES ET LEURS PROCEDES DE PRODUCTION

(30) Priority: 07.11.2002 US 425352 P; 27.05.2003 US 473743 P
(43) Date of publication of application: 17.08.2005
(60) Divisional application: 09012671.5
(73) Proprietor: Kosan Biosciences, Inc., Hayward, CA 94545 (US)
(72) Inventor: LI, Yong, Palo Alto, CA 94306 (US); SUNDERMANN, Kurt, Burlingame, CA 94010 (US); TANG, Li, Foster City, CA 94404 (US); MYLES, David, Kensington, CA 94707 (US)
(74) Representative: Beacham, Annabel Rose
(86) International application number: PCT/US2003/035411
(87) International publication number: WO 2004/043954

(56) References cited:
- WO-A-01/92255
- WO-A-03/022844
- WO-A-2004/043954
- DE-A- 19 907 480
- DE-A- 19 954 229
- J. D. WHITE ET. AL.: "Total Synthesis of Epothilone B, Epothilone D, and cis- and trans- 9,10-Dehydroepothilone D" JOURNAL OF THE AMERICAN CHEMICAL SOCIETY, vol. 123, 2001, pages 5407-13, XP001180980 cited in the application
- M. S. ERMOLENKO ET. AL.: "Synthesis of Epothilones B and D from D-Glucose" TETRAHEDRON LETTERS, vol. 43, 2002, pages 2895-8, XP004345923
- J. D. WHITE ET. AL.: "A Highly Stereoselective Synthesis of Epothilone B." JOURNAL OF ORGANIC CHEMISTRY, vol. 64, 1999, pages 684-5, XP001180981
- A. RIVKIN ET. AL.: "Complex Target-Orientated Total Synthesis in the Drug Discovery Process. The Discovery of a Highly Promising Family of Second Generation Epothilones." JOURNAL OF THE AMERICAN CHEMICAL SOCIETY, vol. 125, 2003, page 2899-901, XP001180982
- JIAN SUN ET. AL.: "Stereoselective Total Synthesis of Epothilones by the Metathesis Approach Involving C9-C10 Bond Formation." ANGEWANDTE CHEMIE, vol. 114, no. 8, 15 April 2002 (2002-04-15), pages 1439-41, XP002295633
- J. D. WHITE ET. AL.: "Synthesis, Conformational Analysis, and Bioassay of 9,10-Didehydroepothilone D." ORGANIC LETTERS, vol. 4, no. 6, 21 March 2002 (2002-03-21), pages 995-7, XP002295634

## Description

### FIELD OF THE INVENTION

The present invention concerns methods for making *trans-9,10-dehydroepothilone* C and D, and analogs thereof, and to compounds made by the methods, compositions containing the compounds and methods for the treatment of hyperproliferative diseases.

### BACKGROUND OF THE INVENTION

The class of polyketides known as epothilones has emerged as a source of potentially therapeutic compounds having modes of action similar to paclitaxel (Bollag, et al., Cancer Res. 55:2325-2333 (1995); Service, Science 274(5295):2009 (1996); Cowden and Paterson, Nature 387(6630):238-9 (1997)). Interest in the epothilones and epothilone analogs has grown with the observations that certain epothilones are active against tumors that have developed resistance to paclitaxel as well as reduced potential for undesirable side-effects (Muhlradt and Sasse Cancer Res. 57(16):3344-6 (1997)). Among the epothilones and epothilone analogs being investigated for therapeutic efficacy are epothilone B **1** and the semi-synthetic epothilone B analogs, BMS-247550 **2**, also known as "azaepothilone B" (Colevas, et al., Oncology (Huntingt). 15(9):1168-9, 1172-5 (2001); Lee, et al., Clin Cancer Res. 7(5):1429-37 (2001); McDaid, et al., Clin Cancer Res. 8(7):2035-43 (2002); Yamaguchi, et al., Cancer Res. 62(2):466-71 (2002)), and BMS-310705 **3**.

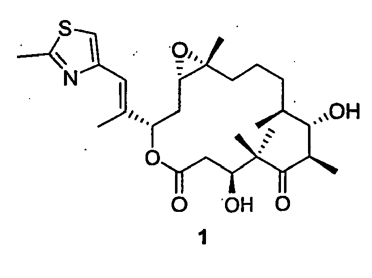

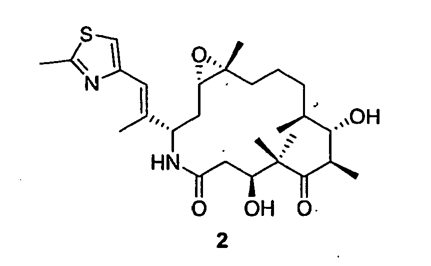

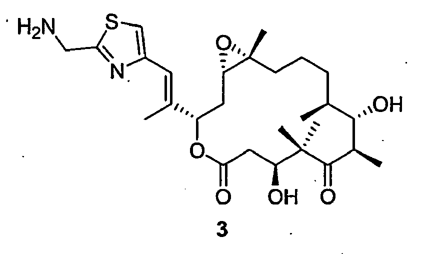

Desoxyepothilone B **4,** also known as "epothilone D" is another epothilone derivative having promising anti-tumor properties viz. paclitaxel that is being investigated for therapeutic efficacy. This compound has also demonstrated less toxicity than epothilones having 12,13-epoxides, such as epothilone B or BMS-247550, presumably due to the lack of the highly reactive epoxide moiety.

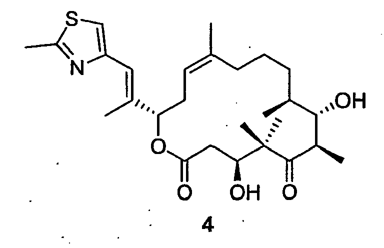

The production of 9-oxo-epothilone analogs, including 9-oxo-epothilone D and analogs thereof using chemical and biotechnological routes, has recently been disclosed in International PCT application Publication No. WO 01/83800, published November 8, 2001.

Recently, the synthesis and preliminary evaluation of *trans*-9,10-dehydroepothilone D (**5**) and 26-trifluoro-*trans*-9,10-dehydroepothilone D (**6**) have been reported (Rivkin et al., J. Am. Chem. Soc. 2003,125: 2899-2901. An earlier report of the preparation of **5** has been found to be in error (White et al., J. Am. Chem. Soc. 2001, 123:5407-13; White et al., J. Am. Chem. Soc. 2003, 125: 3190). While these compounds show promising activity, their preparation by total chemical synthesis is lengthy and expensive, and improved methods for their preparation are needed.

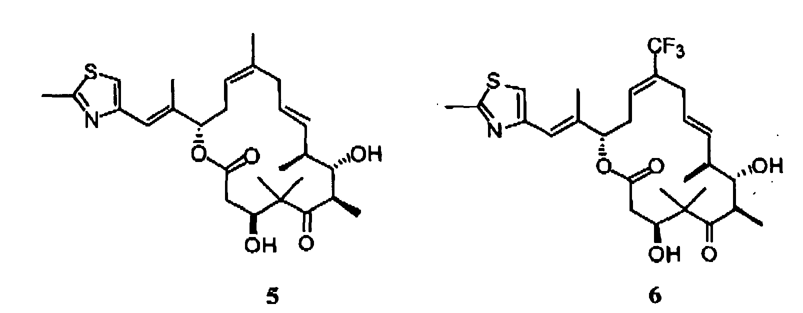

DE-A-19907480; DE-A-19954229; J. Am. Chem. Soc. 123, 5407-13 (2001); Tetrakedron Letters, 43, 2895-8 (2002); J. Or. Chem., 64, 684-5 (1999); J. Am. Chem. Soc., 125, 2899-2901 (2003); Angewandte Chemie, 114,1439-41 (2002); and Organic Letters, 4, 995-7 (2002) describe various 9,10-dehydro-epothilone derivatives which are structurally different from those of the present invention.

Although various epothilone analogs having anti-tumor activity have been disclosed in the art, there is continuing interest in new analogs having microtubule stabilizing activities that exhibit fewer side effects than paclitaxel or epothilones A and B.

There are described herein compounds having microtubule stabilizing activity and useful in the treatment of cancer and other diseases characterized by cellular hyperproliferation of the following formula (I): 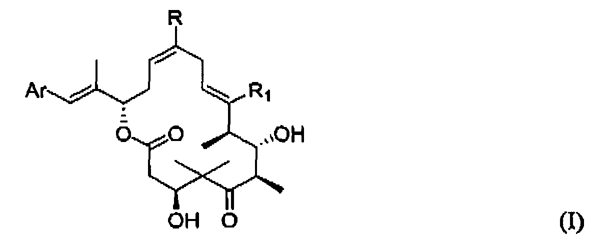 wherein R is H, substituted or unsubstituted loweralkyl; R₁ is H, or is substituted or unsubstituted loweralkyl, loweralkenyl or loweralkynyl;
Ar is substituted or unsubstituted heteroaryl;
and the pharmaceutically acceptable salts or prodrugs thereof; provided that when R is methyl or trifluoromethyl and Ar is 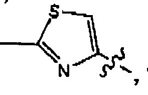 then R₁ is not H.

In some embodiments of the invention, Ar is 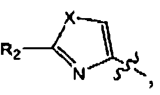 forming compounds of the following structure (II): 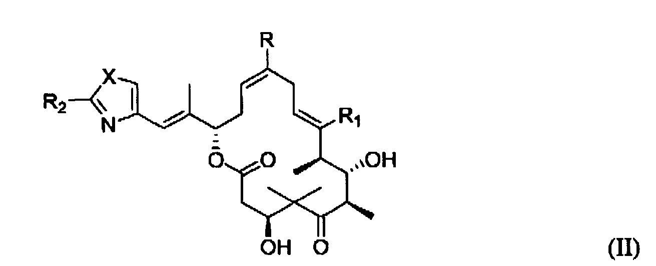 wherein X is S or O, and R₂ is H or substituted or unsubstituted loweralkyl. In other embodiments, Ar may be other substituted or unsubstituted moieties such as, for example substituted or unsubstituted thiazolyl, oxazolyl, imidazolyl, isothiazolyl, isoxazolyl, pyrazolyl, pyridyl, pyrazinyl, pyrimidinyl, pyridazinyl, indolizinyl, indolyl, indazolyl, purinyl, quinolyl, quinolizinyl, phthalazinyl, naphthyridinyl, quinoxalinyl, quinazolinyl, benzothiazolyl, oxadiazolyl, thiadiazolyl, benzotriazolyl, and the like. Presently particularly preferred and novel compounds of the invention are compounds of formula (I) wherein R is methyl or trifluoromethyl and Ar is 2-pyridyl, 4-methoxy-2-pyridyl, 5-(hydroxymethyl)-2-pyridyl, or 5-methyl-3-isoxazolyl.

There are described herein compositions comprising an amount of a described compound effective to inhibit cellular hyperproliferation and/or disrupt tubulin activity in a human or animal subject when administered thereto, together with a pharmaceutically acceptable carrier, and methods of inhibiting cellular hyperproliferation and/or disrupting tubulin activity in a human or animal subject, comprising administering to the human or animal subject a cellular hyperproliferation inhibiting or tubulin activity disrupting amount of a compound or composition of the invention.

There are described methods of synthesizing the compounds of the invention from 9-oxo-epothilone C or D. Also included in the present invention are stents coated with a compound or composition of the invention as described above, and methods of treating cardiovascular disease using such coated stents.

It has now been surprisingly discovered that tubulin activity can be disrupted *in vitro* or *in vivo* by certain *trans*-9,10-dehydroepothilone C and *trans*-9,10-dehydro-epothilone D based derivatives. Accordingly, the present invention provides new compounds, compositions and methods of inhibiting cellular hyperproliferation and/or stabilizing microtubules *in vitro* and of treatment of hyperproliferative diseases *in vivo.*
In one aspect, the present invention provides new compounds of the following formula (I): 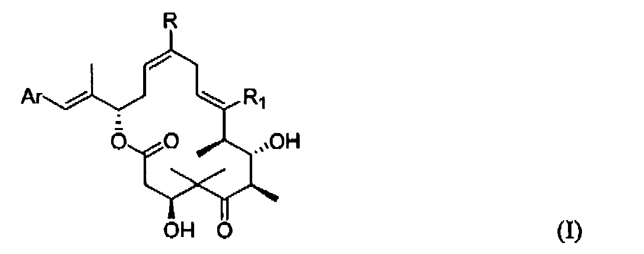 wherein R is H, C₁-C₄ loweralkyl or C₁-C₄ loweralkyl substituted with one or more groups independently selected from halo, hydroxyl, amino and azido;
R₁ is H, or is substituted or unsubstituted C₁-C₄ loweralkyl, C₂-C₄ loweralkenyl, or C₂-C₄ loweralkynyl, which may be substituted with one or more groups independently selected from halo, hydroxyl, amino and azido; and
Ar is 3-isoxazolyl, which may be unsubtituted, monosubstituted or disubustituted with groups independently selected from hydroxy, hydroxyC₁-C₄alkyl, halo, cyano, oxo, alkylimino, amino, alkylamino, dialkylamino, acylaminoalkyl, C₁-C₄ alkoxy, thioalkoxy, C₁-C₄ loweralkyl, cycloalkyl or C₁-C₄ haloalkyl;
and the pharmaceutically acceptable salts or prodrugs thereof.

In some embodiments of the invention, the compounds are of the following structure (II): 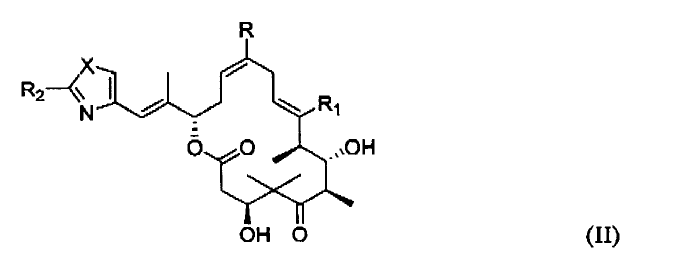 wherein X is O, and R₂ is H, or is substituted or unsubstituted loweralkyl. In certain embodiments, R₂ is substituted methyl. In particular embodiments, R₂ is hydroxymethyl, aminomethyl, alkylaminomethyl, dialkylaminomethyl, azidomethyl, or fluoromethyl.

Compounds of formulas (I) or (II) are provided wherein R is CH₃, and R₁ is H or C₁-C₄ alkyl.

Compounds of formulas (I) or (II) are provided wherein R is H, and R₁ is H or C₂-C₄ alkenyl.

Compounds of formulas (I) or (II) are provided wherein R is CH₃, and R₁ is H or C₂-C₄ alkenyl.

Compounds of formulas (I) or (II) are provided wherein R is H, and R₁ is H or C₂-C₄ alkynyl.

Compounds of formulas (I) or (II) are provided wherein R is CH₃, and R₁ is H or C₂-C₄ alkynyl.

In other embodiments, the invention provides compounds of formula (I) having the structures:

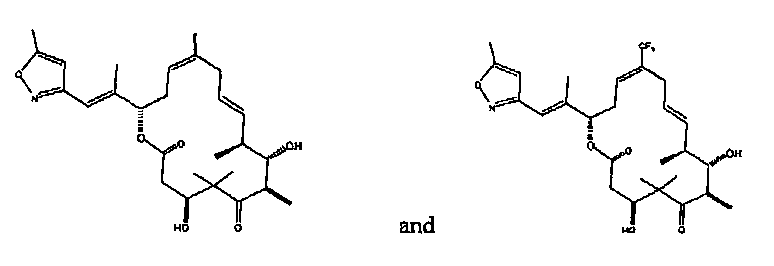

In another aspect, the invention provides compositions comprising an amount of a compound of formula (II) effective to inhibit cellular hyperproliferation and/or disrupt tubulin activity in a human or animal subject when administered thereto, together with a pharmaceutically acceptable carrier.

In yet other embodiments, the invention provides methods of inhibiting cellular hyperproliferation and/or disrupting tubulin activity in a human or animal subject, comprising administering to the human or animal subject a tubulin activity disrupting amount of a compound of formula (II).

The present invention further provides the use of a compound of formula (II) in the manufacture of a medicament for treating human or animal subjects suffering from a hyperproliferative disease, such as cancer.
The medicine may be to the human or animal subject a therapeutically effective amount of a compound of formula (II) above, either alone or in combination with other therapeutically active agents.

There is described stents coated with a compound of the invention and methods for treating cardiovascular disease using such coated stents.

The present invention provides compounds of formula (II) as described above, for use as a pharmaceutical, as well as methods of use of those compounds in the manufacture of a medicament for the treatment of hyperproliferative disease.

There is described stents coated with a compound or composition of the invention, and methods for treating cardiovascular disease using such coated stents.

In yet other embodiments, the present invention provides new methods for making compounds of formula (II) as is hereinafter described in detail.

As used herein the following terms have the meanings defined below:

Loweralkyl groups described herein may be branched or straight chain alkyl groups comprising one to four carbon atoms (C₁-C₄ alkyl).

Loweralkenyl groups described herein may be straight chain, branched, or cyclic radicals having one or more double bonds and from two to four carbon atoms (C₂-C₄ alkenyl).

Loweralkynyl groups described herein may be straight chain, or branched having two to four carbon atoms (C₂-C₄ alkynyl).

The loweralkyl, loweralkenyl, or loweralkynyl moieties as defined herein may be substituted or unsubstituted e.g., with one or more halogen, hydroxyl, amino, azido or other groups, including, e.g., methyl, ethyl, propyl, isopropyl, n-butyl, t-butyl, neopentyl, trifluoromethyl, hydroxymethyl, aminomethyl, azidomethyl, pentafluoroethyl and the like.

"Pharmaceutically acceptable salts" useful in the practice of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-napthalenesulfonate, oxalate, pamoate, pectinate, sulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Basic addition salts can be prepared *in situ* during the final isolation and purification of the compounds of formula (I), or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

The term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

There are described methods of synthesizing the compounds of structures (II), (III) or (IV), wherein X is S, from 9-oxo-epothilones C or D. 9-oxo-epothilones C and D, may be readily obtained as disclosed in International PCT application Publication No. WO 01/83800, published November 8, 2001, the disclosure of which is incorporated herein by this reference. In general, 9-oxo-epothilone D is produced by in recombinant host cells of the suborder *Cystobacterineae* containing a recombinant expression vector that encodes a polyketide synthase (PKS) gene. As disclosed in WO 01/83800, inactivation of the KR domain of extender module 6 of the epothilone PKS results in a PKS capable of producing the 9-oxo-epothilones. A strain in which the KR domain of extender module 6 has been deactivated has been designated K39-164 and has been deposited with the American Type Culture Collection, Manassas, Virginia 20110-2209, USA on November 21, 2000, under the terms of the Budapest Treaty, and is available under accession No, PTA-2716. Strain K39-164 produces 9-oxo-epothilone D as a major product and 9-oxo-epothilone C as a minor product. In another aspect, the present invention relates to methods of synthesizing the compounds of structures (II) wherein X is O, from 9-oxo-epothilones H₁ or H₂, the oxazole counterparts of 9-oxo-epothilones C or D. As also described in WO 01/83800, by supplementing host cells with excess serine, the thiazole epothilone compounds normally produced by host cells are modulated in such a way to favor the production of the oxazole counterparts, In this manner, cells that predominantly produce 9-oxo-epothilone C or D can be made to favor the production of 9-oxo-epothilone H₁or H₂, the corresponding oxazole counterparts.

Referring to reaction scheme 1, below, compounds may be obtained by ketoreduction and elimination, such as by protecting the free hydroxyl groups with a suitable protecting group, such as, for example, a triethylsilyl group, a butyldimethylsilyl group, a 2,2,2-trichloroethoxycarbonyl group or the like; reducing the 9-oxo group to obtain the corresponding 9-hydroxyl intermediate compound, activating the 9-hydroxyl compound such as with trifluoroacetic anhydride, Methanesulfonyl chloride or trifluoromethanesulfonic anhydride in the presence of a suitable base, for example sodium bis(trimethylsilyl)amide or an amine base such as pyridine or 4-(dimethylaminopyridine) in tetrahydrofuran ("THF") or other suitable solvent to obtain the corresponding trifluoroacetyl, methanesulfonate or trifluoromethanesulfonate intermediate compound; eliminating the activating group to obtain the corresponding protected 9,10-dehydroepothilone by reaction with a suitable base, for example sodium bis(trimethylsilylamide), lithium diisopropylamide, or 4-(dimethylaminopyridine); and then deprotecting the protected intermediate to obtain the desired 9,10-dehydro-epothilone. Representative examples utilizing this synthesis route are set forth hereinbelow in Examples 1-21.

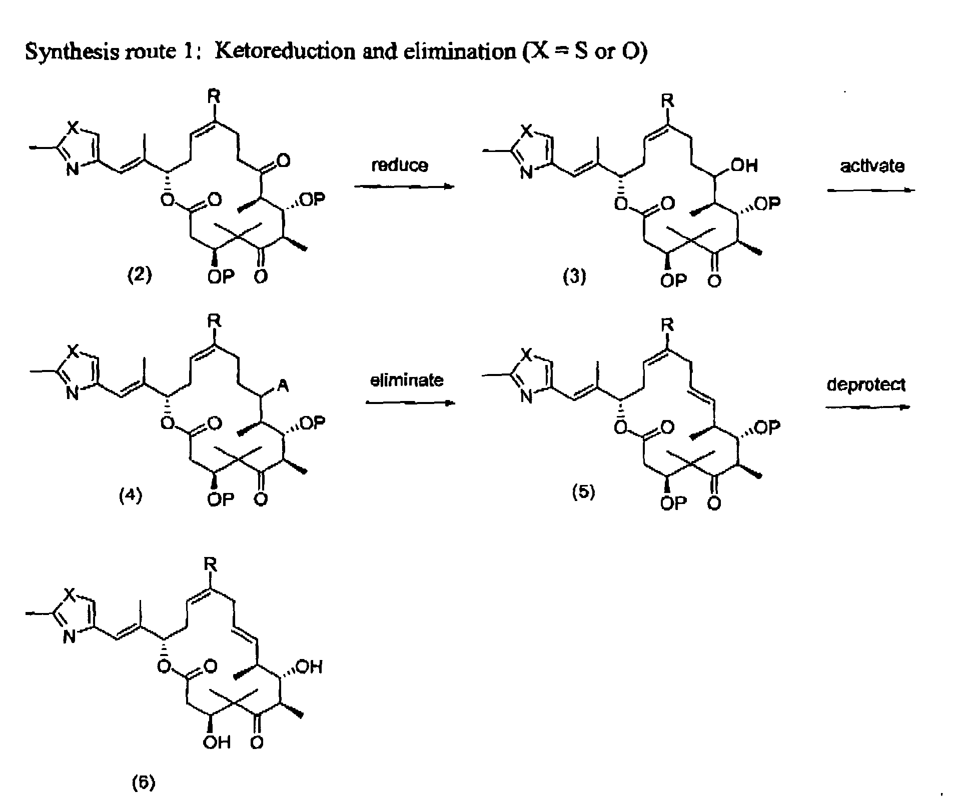

Referring to reaction scheme 2, below, compounds may also be obtained by pyrolytic elimination, such as by activation of the bis(protected) 9-oxo-epothilone with a base, such as sodium bis(trimethylsilyl)amide, carbon disulfide and methyl iodide so as to form the methyl xanthate ester, or with dimethylthiocarbamoyl chloride so as to form the thiocarbamate, and then heating the activated intermediate at a sufficient temperature, such as at about 170 °C, for a sufficient time to eliminate the activating group and obtain the corresponding protected 9,10-dehydro epothilone compound, and then deprotecting the protected intermediate to obtain the desired 9,10-dehydroepothilone. Suitable temperatures and times can be determined using methods familiar to those having skill in the organic chemistry arts. Representative examples utilizing this synthesis route are set forth hereinbelow in Examples 1-4 and 22-25.

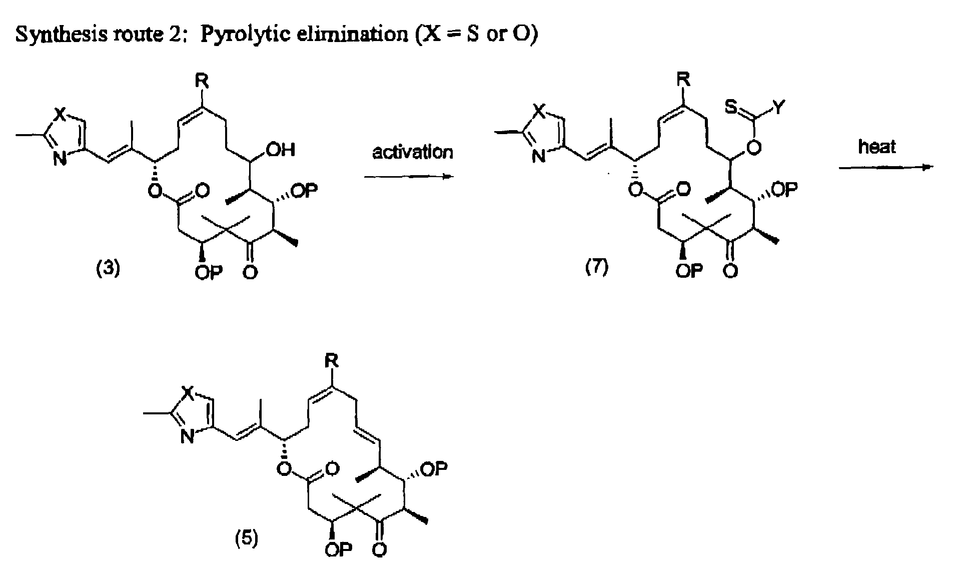

Referring to reaction scheme 3, below, compounds may also be obtained by vinyl triflate reduction, such as by reacting a protected 9-oxo-epothilone with a triflating reagent such as trifluoromethanesulfonic anhydride, N-(2-pyridyl)triflimide, or N-(5-chloro-2-pyridyl)triflimide, and a base such as 2,4-di-tert-butyl-4-methylpyridine, or sodium bis(trimethylsilyl)amide to obtain the corresponding 9,10-dehydro-9-trifluoromethanesulfonyloxy intermediate compound. The 9-trifluoromethanesulfonyloxy group may then be reduced, for example using a hydride source such as a trialkylammonium formate salt or a trialkyltin hydride in the presence of a palladium catalyst, such as tetrakis(triphenylphosphine)palladium(0), to form the resulting protected 9,10-dehydro-epothilone, and the compound deprotected to obtain the desired 9,10-dehydroepothilone **10**. Alternatively, the 9,10-dehydro-9-trifluoromethanesulfonyloxy intermediate compound may be further substituted by the addition of alkyl groups using, for example, vinyl triflate coupling techniques, such as reaction of the trifluoromethylsulfonyloxy enol (8) with allyltributyltin in the presence of a catalyst such as tetrakis(triphenylphosphine)-palladium(0) and lithium chloride to obtain the allyl-substituted, protected 9,10-dehydro epothilone compound **9a** (R₁ = CH₂CHCH₂); and then deprotecting the protected 9-substituted intermediate to obtain the desired 9-substituted, 9,10-dehydroepothilone **10a**. In a similar manner, reaction of intermediate (8) with loweralkyl zinc reagents in the presence of a catalyst such as (tetrakis)triphenylphosphinepalladium(0) provides compounds of formula (9a) wherein R1 is loweralkyl. Representative examples utilizing these synthesis routes are set forth hereinbelow in Examples 1-4 and 26-30.

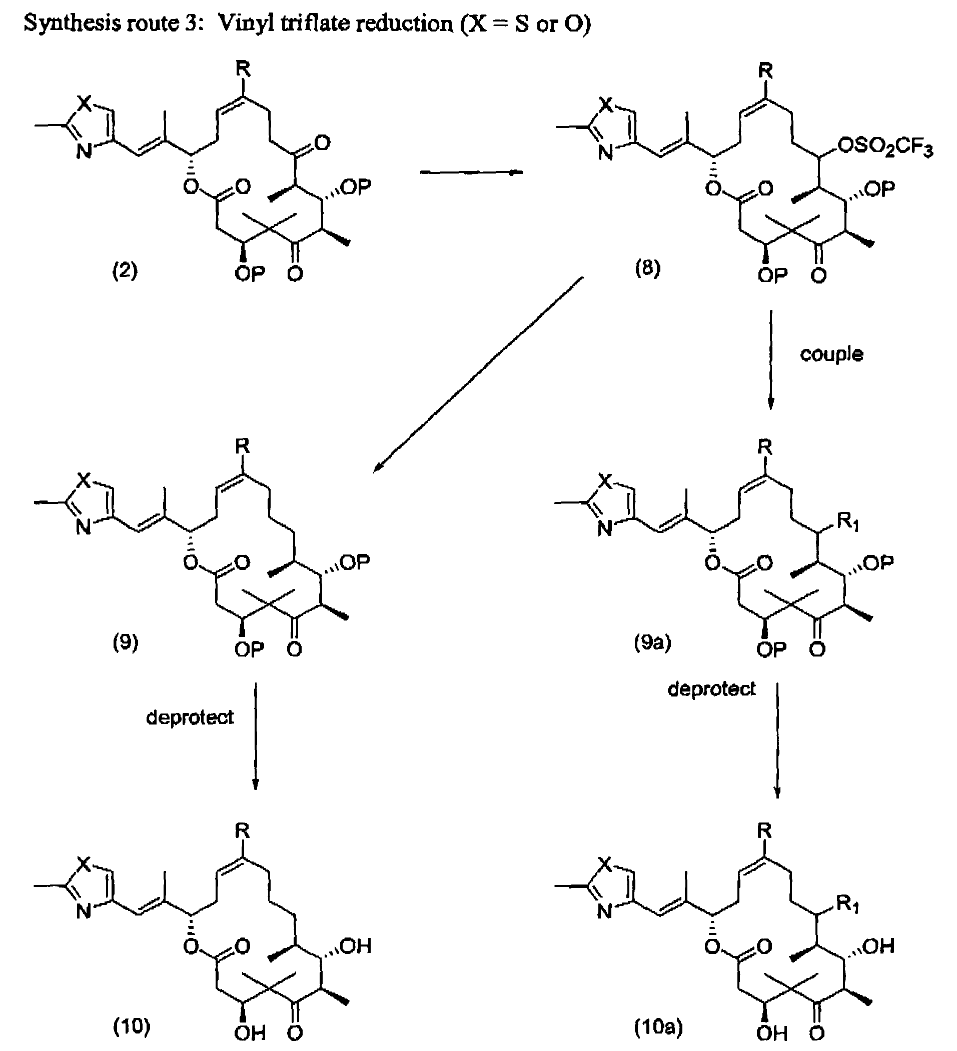

Referring to reaction scheme 4, below, compounds may also be prepared from 9-oxoepothilones using ring-opened intermediates. In one embodiment of the invention, a 3,7-protected form of the epothilone (2) is reacted with a reducing agent, for example di(isobutyl)aluminum hydride (DiBA1-H) under conditions
wherein the lactone carbonyl is selectively reduced. The alcohol groups on the resulting ring-opened intermediate (11) are protected, for example by silylation using a chlorotrialkylsilane or trialkylsilyl triflate in the presence of a base such as imidazole or 2,6-lutidine, to provide intermediate (12). In another embodiment of the invention, a 3,7-protected form of the epothilone is reacted with a reducing agent, for example di(isobutyl)aluminum hydride (DiBAl-H) under conditions wherein both the lactone carbonyl and the 9-oxo group are reduced to provide intermediate. The 1- and 15-OH groups are selectively protected, for example as their tert-butyldimethylsilyl ethers by reaction with their tert-butyldimethylsilyl chloride and imidazole, to provide intermediate (13). The 9-OH may be oxidized back to the ketone using, for example, the Dess-Martin periodinane in dichloromethane, to provide intermediate (12).

The ketone group of intermediate (12) can be transformed into a trans-9,10-alkene using any of the methods described above. In one embodiment, intermediate (12) is reacted with a triflating agent such as trifluoromethanesulfonic anhydride or an N-(2-pyridyl)triflimide and a base, such as sodium bis(trimethylsilyl)amide or lithium diisopropylamide, to form the vinyl triflate (14). The vinyl triflate is reduced using a hydride source such as a trialkylammonium formate salt or trialkyltin hydride in the presence of a catalyst such as tetrakis(triphenylphosphine)palladium(0) to provide intermediate (15).

Intermediate (13) may be reacted with a reagent to activate the 9-group for elimination. For example, (13) is reacted with trifluoroacetic anhydride, methanesulfonic anhydride, or trifluoromethanesulfonic anhydride in the presence of a suitable base, such as pyridine or 4-(dimethylaminopyridine), to produce the 9-0-trifluoroacetate, 9-O-methanesulfonate, or 9-O-triflate, respectively. Subsequent treatment with a hindered base, for example sodium bis(trimethylsilyl)amide or lithium diisopropylamide produces the trans-9,10-alkene intermediate (15).

Intermediate (13) may be converted to the methyl xanthate by reaction with a base such as sodium bis(trimethylsilyl)amide, carbon disulfide, and methyl iodide. The intermediate xanthate is subjected to pyrolysis as described above to produce intermediate (15).

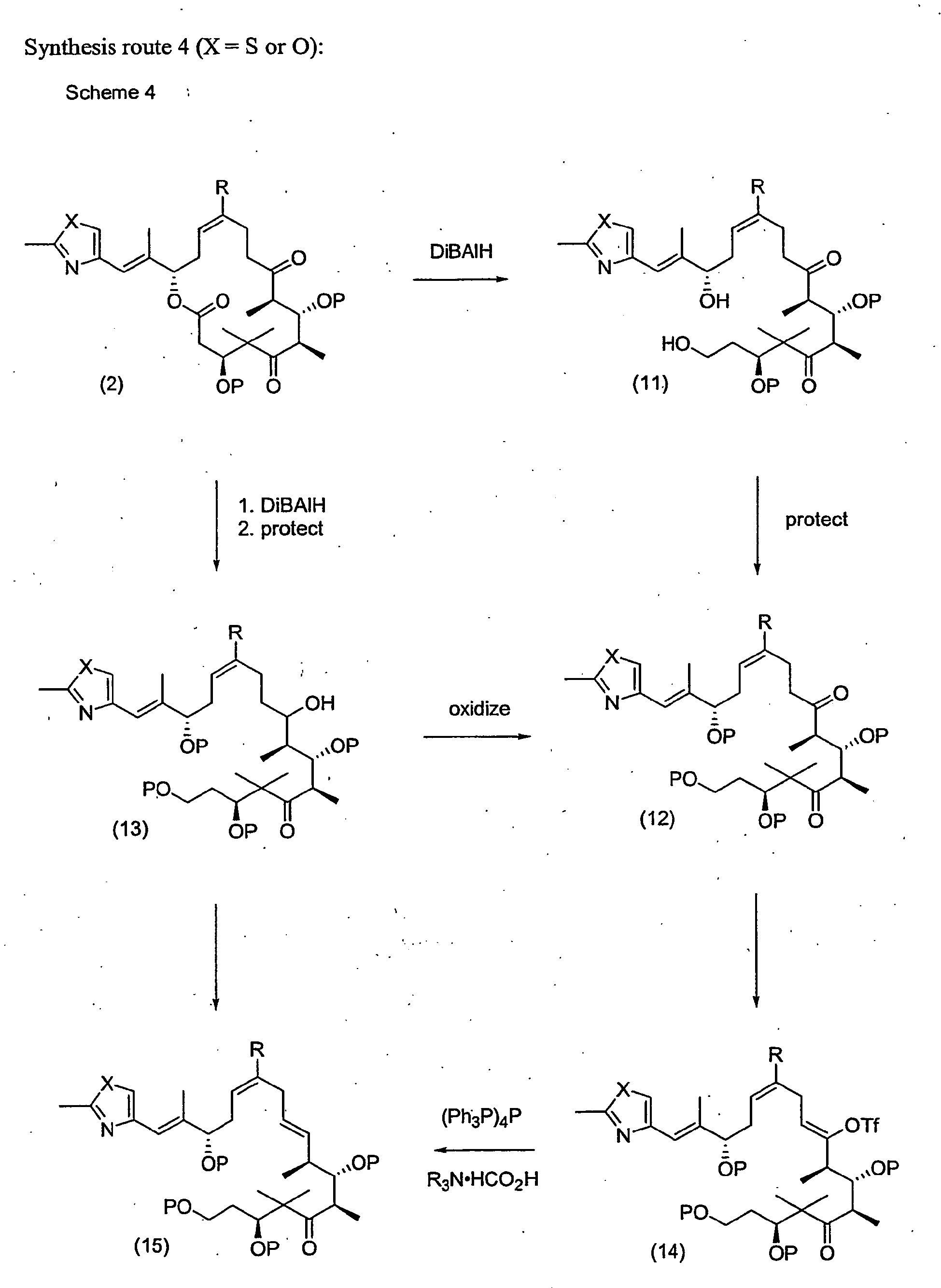

Intermediate (15) can be converted into trans-9,10-dehydroepothilones. The primary silyl protecting group is removed using, for example, camphorsulfonic acid, and the resulting 1-alcohol is oxidized to the carboxylic acid, for example using a two-step procedure wherein the alcohol is first oxidized using an amine oxide and catalytic tetrapropylammonium perruthenate and then further oxidized to the acid by reaction with sodium chlorite (NaClO₂). The 15-OH group is deprotected by treatment with tetrabutylammonium fluoride in THF at 0 °C, and the macrocycle is closed, for example using the conditions of Yamaguchi (1,3,5-trichlorobenzoyl chloride and triethylamine in THF at 0 °C, followed by addition of the mixed anhydride to a solution of 4-(dimethylamino)pyridine in toluene at 75°C) to provide the protected intermediate (13). Deprotection provides the trans-9,10-dehydroepothilone.

Referring to synthesis scheme 5, compounds can be prepared using ring-opened epothilone intermediates prepared by saponification. In one embodiment, the protected epothilone (2) is converted into the protected seco-acid, for example by treatment with hydroxide or an esterase. The acid is converted to the tert-butyl ester, for example using tert-butyl alcohol, a carbodiimide such as dicyclohexylcarbodiimide, and 4-(dimethylamino)pyridine as catalyst. The 15-OH is protected, for example by silylation, and the 9-ketone is converted to the vinyl triflate as described above. The vinyl triflate is reduced as described above, and the intermediate is partially deprotected using camphorsulfonic acid to provide the 3,7-protected-seco-9,10-dehydroepothilone. Lactonization according to the procedure of Yamaguchi followed by deprotection provides the trans-9,10-dehydroepothilone.

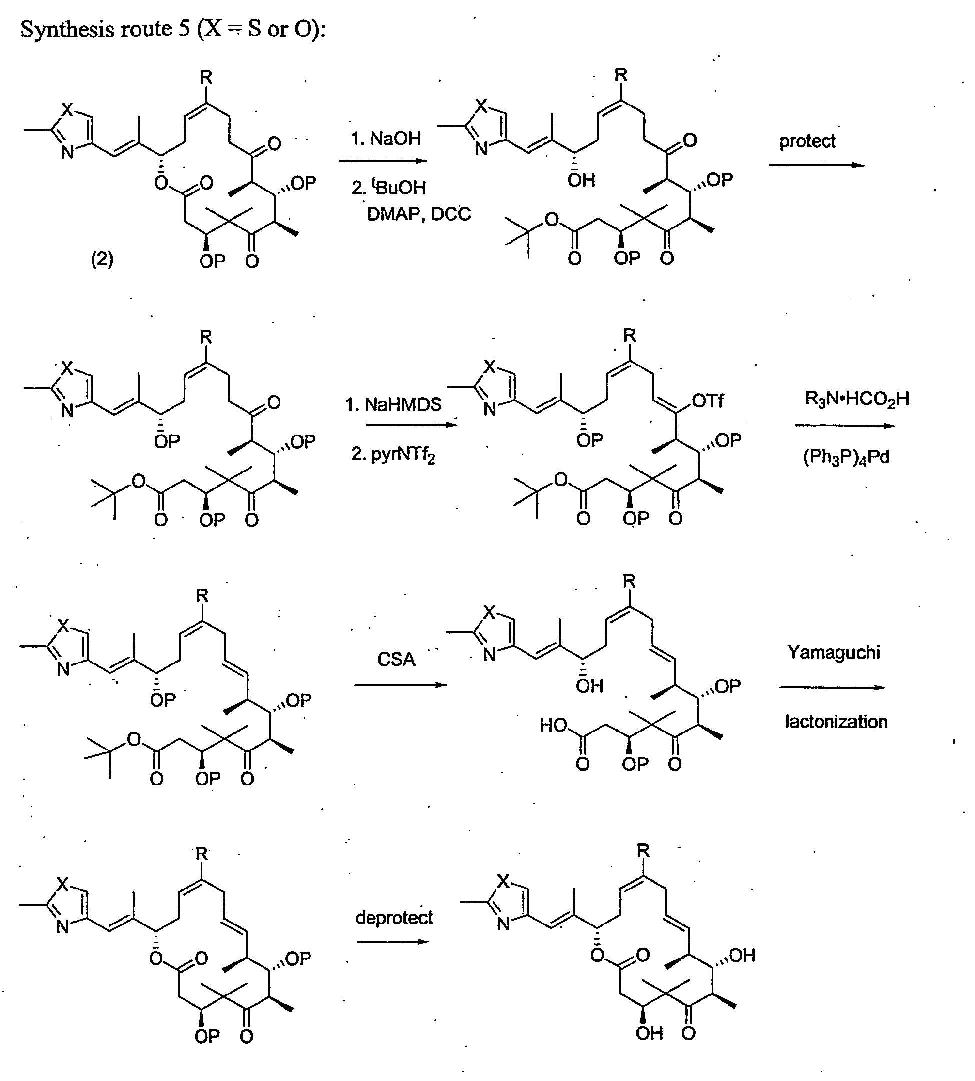

The 3,7-protected form of epothilones (2) can be converted into trans-9,10-dehydroepothilones. The synthesis route involves initial saponification of epothilone derivative (2) to provide a ring-opened epothilone intermediate, which is modified to include the 9,10-trans alkene group and then ultimately lactonized to provide the trans-9,10-dehydroepothilone. A representative synthetic scheme is illustrated in synthesis route 6.

Referring to synthesis route 6, the 3,7-protected epothilone (2) is saponified with sodium hydroxide in aqueous methanol to provide the corresponding hydroxy acid (16). Hydroxy acid (16) is converted to its methyl ester (17) by reaction with trimethylsilyl diazomethane. The hydroxy group of methyl ester (17) is protected by treatment with trimethylsilyl chloride/trimethylsilyl imidazole to provide trimethylsilyl ether (18). The 9-ketone of trimethylsilyl ether (18) is transformed into a trans-9,10-alkene by reaction with N-(2-pyridyl)triflimide and sodium bis(trimethylsilyl)amide to produce vinyl triflate (19). Reduction of vinyl triflate (19) with palladium (II) acetate, triphenylphosphine, tributylamine, and formic acid provides trans-9,10-alkene (20). The trimethylsilyl protecting group and methyl ester group of intermediate (20) are removed by treatment with sodium hydroxide followed by treatment with acetic acid yielding hydroxy acid (21), which is then lactonized to provide 3,7-protected epothilone (9). Deprotection provides the trans-9,10-dehydroepothilone.

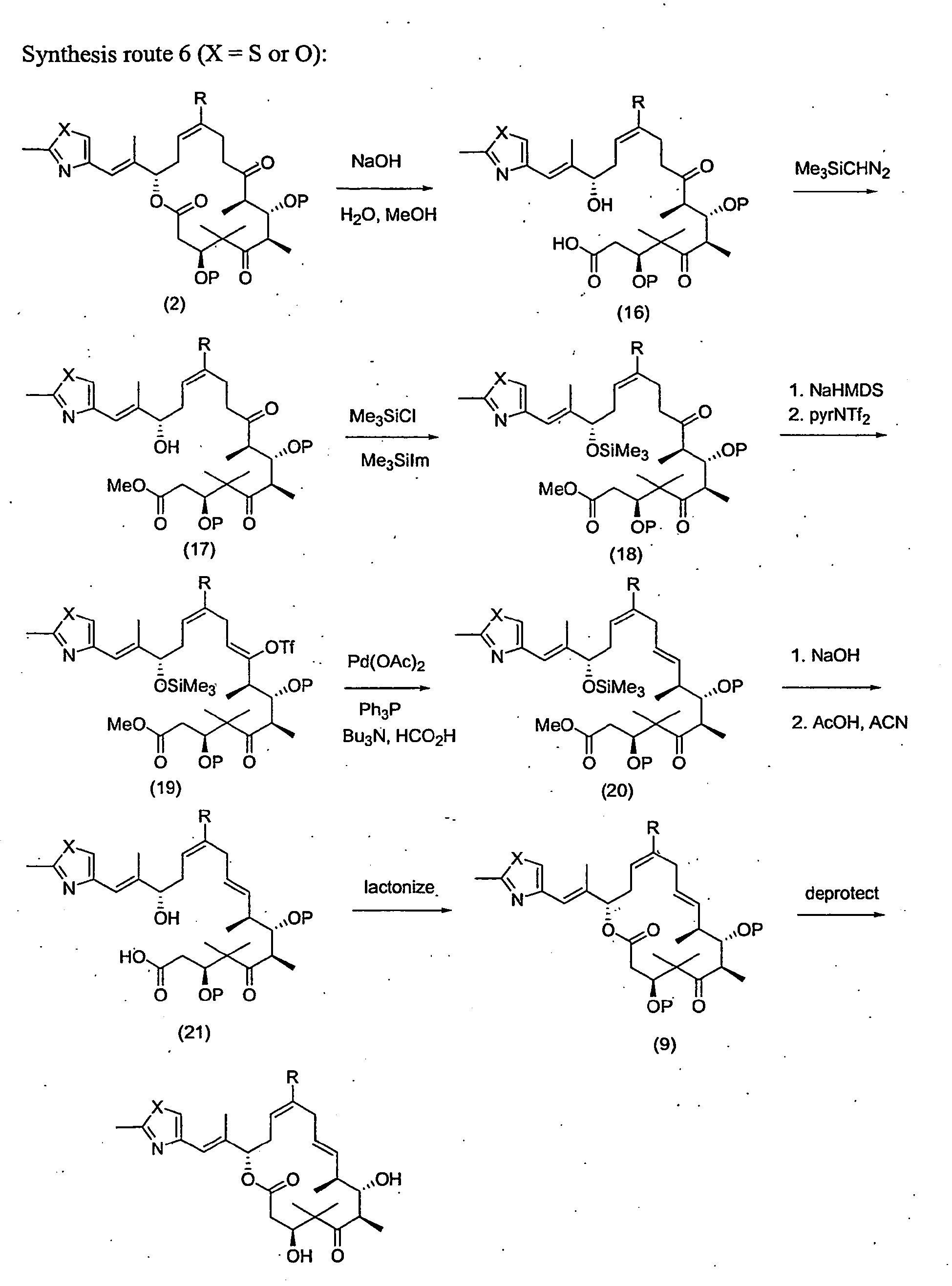

Ring-opened trimethylsilyl ether (18) prepared as described above and as shown in synthesis route 6 can be the starting point for another route to trans-9,10-dehydroepothilones. The synthetic route involves reduction of the 9-ketone group of intermediate (18) followed by elimination to provide the trans-9,10-alkene group and then ultimately lactonization to provide the trans-9,10-dehydroepothilone. A representative synthetic scheme is illustrated in synthesis route 7.

Referring to synthesis route 7, reduction of trimethylsilyl ether (18) with sodium borohydride provides 9-hydroxy derivative (22). Hydroxy derivative (22) is then activated for elimination by treatment with an activating agent, such as trifluoroacetic anhydride, methanesulfonic anhydride, or trifluoromethanesulfonic anhydride in the presence of a suitable base, such as pyridine or 4-(dimethylaminopyridine), to produce the 9-O-trifluoroacetate, 9-O-methanesulfonate, or 9-O-triflate, respectively, activated intermediate (23). Subsequent treatment of activated intermediate (23) with a hindered base, for example, sodium bis(trimethylsilyl)amide or lithium diisopropylamide produces the trans-9,10-alkene intermediate (24). The trimethylsilyl protecting group and methyl ester group of intermediate (24) are removed by treatment with sodium hydroxide followed by treatment with acetic acid to yield hydroxy acid (21).Lactonization of hydroxy acid (21) provides 3,7-protected epothilone (9), which on deprotection yields the trans-9,10-dehydroepothilone.

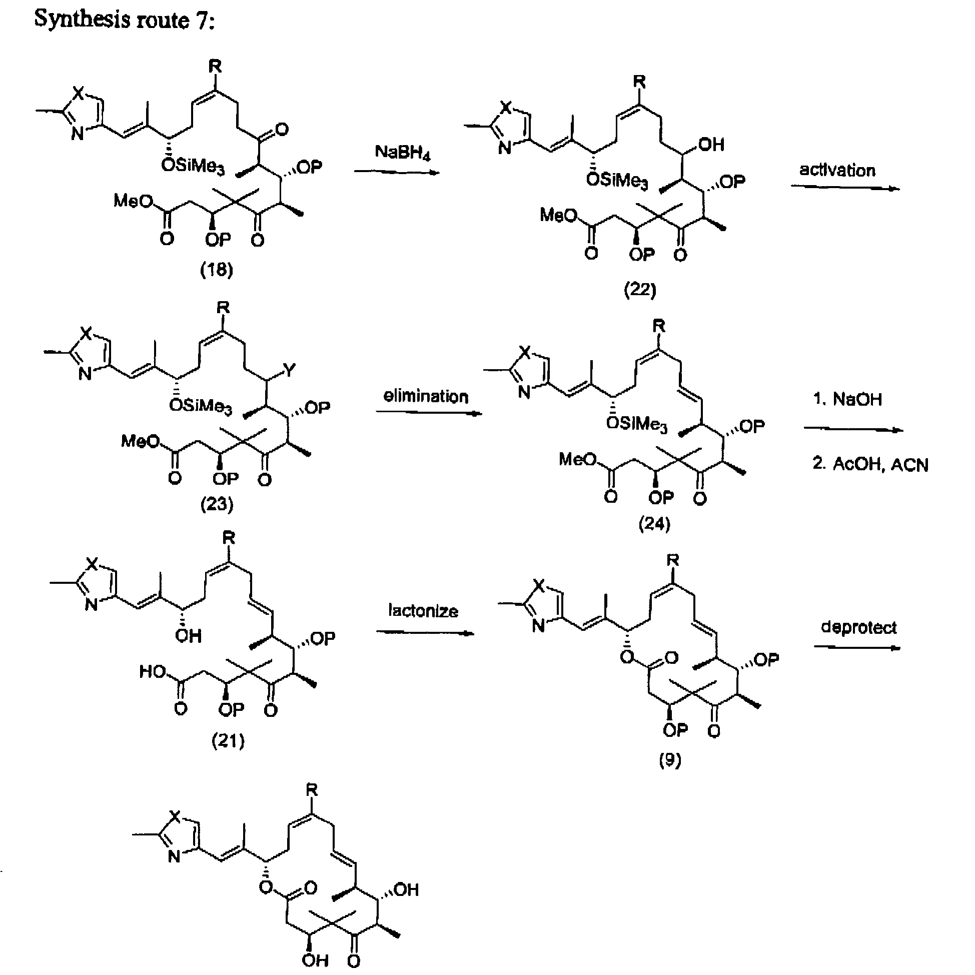

Compounds may be prepared by total synthesis as illustrated in Synthesis Route 8. Ketone (25), wherein P₁ and P₂ are hydroxyl protecting groups, may be prepared as described, for example, in A. Rivkin et al., J. Am. Chem. Soc. 2003 125: 2899-2901. Typical hydroxyl protecting groups include silyl ethers, for example trimethylsilyl (TMS), triethylsilyl (TES), and tert-butyldimethylsilyl (TBS); esters, for example acetate, chloroacetate, trifluoroacetate, or benzoate; carbonates, for example methyl carbonate, trichloroethyl carbonate (troc), or allyl carbonate (alloc); and acetals, for example methoxymethyl (MOM) or benzyloxymethyl (BOM). In particular embodiments, P₁ and P₂ are silyl ethers. Reaction of (25) with suitable Wittig ylids yields protected compounds (26). Subsequent deprotection as described in the Examples below provides the compounds of formula (I). The Wittig ylids may be prepared by reaction of the corresponding phosphonium salts with a strong base, for example sodium bis(trimethylsilyl)amide (NaHMDS), potassium bis(trimethylsilyl)amide (KHMDS), lithium diisopropylamide (LDA), butyllithium, sodium hydride, or similar. The phosphonium salts may be prepared by reaction of the alkyl halides with a triaryl- or trialkyl-phosphine, for example triphenylphosphine or tributylphosphine. In particular embodiments of the invention, the phosphonium salts are prepared by reaction of alkyl chlorides with tributylphosphine. Alternatively, Wittig ylids may be prepared according to other methods known in the art, for example by treatment of alkyldiphenylphosphine oxides with a strong base. Examples of the preparation of various phosphonium salts suitable for use in the preparation of compounds of formula (I) are provided in the Examples below.

Deprotection (removal of P₁ and/or P₂) may be performed using methods known in the art, for example as described in Greene and Wuts, Protective Groups in Organic Synthesis, 3rd Edition (Wiley, New York), 1999. In particular embodiments, when P₁ and P₂ are silyl ethers such as TMS, TES, or TBS, deprotection is performed by treatment with acid, for example HF·pyridine or trifluoroacetic acid in dichloromethane.

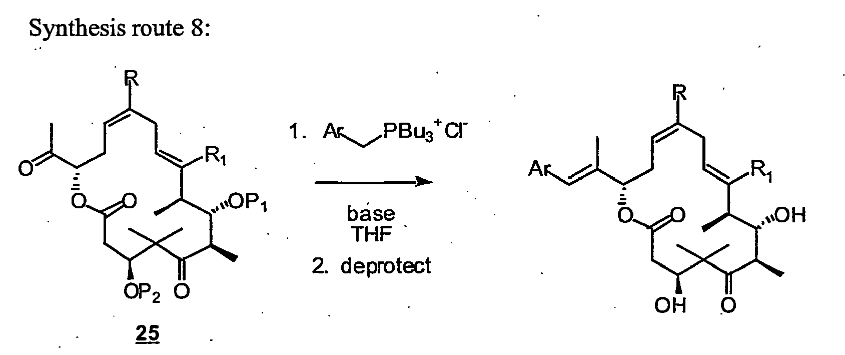

Compounds of the invention may be screened for cytotoxicity using conventional assays well known to those skilled in the art. For example, cytotoxicity of the compounds may be determined by the SRB assay as disclosed in Skehan et al., J. Natl. Cancer Inst. 82:1107-1112 (1990), which is incorporated herein by reference. In the SRB assay, the following cultured cells are trypsinized, counted and diluted to the following concentrations per 100 µl with growth medium: MCF-7, 5000; NCI/ADR-Res, 7500; NCI-H460, 5000; A549, 5000; Skov3, 7500; and SF-268, 7500. The cells are seeded at 100 µl/well in 96-well microtiter plates. Twenty-four hours later, 100 µl of a test epothilone compound of the invention (ranging in concentration from 1000 nM to 0.001 nM diluted in growth medium) is added to each well. After incubation, with the compound for a period of days, the cells are fixed with 100 µl of 10% trichloroacetic acid ("TCA") at 4 degrees for 1 hour, and stained with 0.2% sulforhodamine B (SRB)/1% acetic acid, and the bound SRB is then extracted with 200 µl of 10 mM Tris base. The amount of bound dye is determined by OD 515 nm, which correlates to the total cellular protein contents. The data is analyzed using standard statistical methodologies (such as those available with the software sold as KALEIDA GRAPH^{®} by Synergy Software of Reading, PA) and the IC₅₀s are calculated.

Cytotoxicity results for carious compounds of formula (I) are given in Tables 1 and 2 below. As can be seen, compounds of the invention can unexpectedly show cytotoxicity activity as good as or superior to trans-9,10-dehydroepothilone D.

Compounds of the invention may also be screened for tubulin polymerization using conventional assays well known to those skilled in the art. For example, the compounds may be assayed for tubulin polymerization using the procedure of Giannakakou et al., J. Biol. Chem. 271:17118-17125 (1997) and/or Intl. J. Cancer 75:57-63 (1998), which are incorporated herein by reference. In this procedure, MCF-7 cells are grown to confluency in 35mm culture dishes and treated with 1 nM of a compound of the invention for 0, 1 or 2 hours at 35 °C. After washing the cells twice with 2 ml of phosphate buffered saline (PBS) without calcium or magnesium, the cells are lysed at room temperature for 5-10 minutes with 300 µl of lysis buffer (20 mM Tris, PH 6.8, 1 mM MgCl2, 2 mM EDTA, 1% Triton X-100,plus protease inhibitors). The cells are scraped and the lysates transferred to 1.5 ml Eppendorf tubes. The lysates are then centrifuged at 18000 g for 12 minutes at room temperature. The supernatant containing soluble or unpolymerized (cytosolic) tubulin is' separated from the pellet containing insoluble or polymerized (cytoskeletal) tubulin and transferred to new tubes. The pellets are then resuspended in 300 µl of lysis buffer. Changes in tubulin polymerization in the cells are determined by analyzing the same volume of aliquots of each sample with SDS-PAGE, followed by immunoblotting using an anti-tubulin antibody (Sigma).

In other aspects, the present invention relates to compositions comprising a compound of the present invention and a pharmaceutically acceptable carrier. The inventive compound may be free form or where appropriate as pharmaceutically acceptable derivatives such as prodrugs, and salts and esters of the inventive compound.

Compositions of the invention may be in any suitable form such as solid, semisolid, liquid or aerosol form. See Pharmaceutical Dosage Forms and Drug Delivery Systems, 5th edition, Lippicott Williams & Wilkins (1991) which is incorporated herein by reference. In general, the pharmaceutical comprise one or more of the compounds of the invention as an active ingredient in admixture with an organic or inorganic carrier or excipient suitable for external, enteral, or parenteral application. The active ingredient may be compounded, for example, with conventional non-toxic, pharmaceutically acceptable carriers for tablets, pellets, capsules, suppositories, pessaries, solutions, emulsions, suspensions, and other forms suitable for use. Pharmaceutically acceptable carriers for use in the compositions include, for example, water, glucose, lactose, gum acacia, gelatin, mannitol, starch paste, magnesium trisilicate, talc,corn starch, keratin, colloidal silica, potato starch, urea, and other carriers suitable for use in manufacturing preparations, in solid, semi-solid, liquified or aerosol form. The compositions may additionally comprise auxiliary stabilizing, thickening, and/or coloring agents and perfumes.

In one embodiment, the compositions comprising a compound of the invention are Cremophor^{®}-free. Cremophor^{®} (BASF Aktiengesellschaft) is a polyethoxylated castor oil which is typically used as a surfactant in formulating low soluble drugs. However, because Cremophor^{®} can case allergic reactions in a subject, compositions that minimize or eliminate Cremophor^{®} are preferred. Formulations of epothilone A or B that eliminate Cremophor^{®} are described for example by PCT Publication WO 99/39694, which is incorporated herein by reference, and may be adapted for use with the compounds of the present invention. For example, compositions may comprise a compound of the invention together with pharmaceutically acceptable carriers such as alcohols (for example, ethanol), glycols (for example, propylene glycol), polyoxyethylene compounds such as polyethyleneglycols (PEG), Tween^{®} (polyoxyethylene sorbitan monoesters; ICI America), and Solutol^{®} (polyethyleneglycol 660 12-hydroxystearate; BASF Aktiengesellschaft), or medium chain triglycerides (Miglyol^{®}, Huls Aktiengesellschaft). In certain embodiments of the invention, compositions comprising a compound of formula (I) together with ethanol, propylene glycol, and Tween-80® are provided. In certain other embodiments of the invention, compositions comprising a compound of formula (I) together with ethanol, propylene glycol, and Solutol HS-15^{®} are provided. Alternatively, Cremophor^{®}-free compositions of the invention may comprise at least one cyclodextrin, and, in more particular embodiments, the cyclodextrin may be a hydroxyalkyl-β-cyclodextrin or sulfoalkyl-β-cyclodextrin, and, in a still more particular embodiment, a hydroxypropyl-β-cyclodextrin or sulfobutyl-β-cyclodextrin. Still more particular embodiments in which the carrier includes a hydroxypropyl-β-cyclodextrin include those for which the hydroxypropyl-β-cyclodextrin has a degree of substitution of at least about 4.6%, and, more specifically a degree of substitution of at least about 6.5%. Still more specific embodiments of the composition of the invention are those for which the carrier comprises a hydroxypropyl-β-cyclodextrin having a degree of substitution between about 4.6% and about 6.5%.

In one embodiment of the invention, the composition comprises a compound of formula (I), an alcohol, a glycol, and a cyclodextrin. In certain embodiments, the composition comprises a compound of formula (I), ethanol, propylene glycol, and a cyclodextrin. In one embodiment, the composition comprises a compound of formula (I), together with about 5% to about 20% ethanol, about 1% to about 10% propylene glycol, and about 5% to about 20% of a β-cyclodextrin. In a particular embodiment, the composition comprises a compound of formula (I), together with about 7% ethanol, about 3% propylene glycol, and about 12% of a β-cyclodextrin.

Where applicable, the inventive compounds may be formulated as microcapsules and/or nanoparticles. General protocols are described for example, by Microcapsules and Nanoparticles in Medicine and Pharmacy, Max Donbrow, ed., CRC Press (1992) and by U.S. Patent Nos. 5,510,118; 5,534,270; and 5,662,883, which are all incorporated herein by reference. By increasing the ratio of surface area to volume, these formulations allow for the oral delivery of compounds that would not otherwise be amenable to oral delivery.

Compositions of the invention may also be formulated using other methods that have been previously used for low solubility drugs. For example, compounds of the invention may be formulated in emulsions with vitamin E or a PEGylated derivative thereof as described by WO 98/30205 and by WO 00/71163, which are incorporated herein by reference. Typically, the compound is dissolved in an aqueous solution containing ethanol (preferably less than 1% w/v), and then vitamin E or a PEGylated-vitamin E is added. The ethanol is then removed to form a pre-emulsion that can be formulated for intravenous or oral routes of administration. Alternatively, compounds of the invention may be encapsulated in liposomes. Methods for forming liposomes as drug delivery vehicles are well known in the art. Suitable protocols include those described by U.S. Patent Nos. 5,683,715; 5,415,869, and 5,424,073, which are incorporated herein by reference, relating to another relatively low solubility cancer drug taxol, and by PCT Publication WO 01/10412, which is incorporated herein by reference, relating to epothilone B. Of the various lipids, that may be used, presently particularly preferred lipids for making epothiloneencapsulated liposomes include, for example, phosphatidylcholine and polyethyleneglycol-derivitized distearyl phosphatidylethanolamine.

In yet other embodiments, compositions of the invention may comprise polymers, such as biopolymers or biocompatible (synthetic or naturally occurring) polymers. Biocompatible polymers can be categorized as biodegradable and non-biodegradable. Biodegradable polymers degrade *in vivo* as a function of chemical composition, method of manufacture, and/or implant structure. Illustrative examples of synthetic polymers include, for example, polyanhydrides, polyhydroxyacids such as polylactic acid, polyglycolic acids and copolymers thereof, polyesters, polyamides, polyorthoesters and some polyphosphazenes. Illustrative examples of naturally occurring polymers include proteins and polysaccharides such as collagen, hyaluronic acid, albumin and gelatin.

In yet other embodiments, compounds of the present invention may be conjugated to a polymer that enhances aqueous solubility. Representative examples of polymers suitable for this purpose include polyethylene glycol, poly-(d-glutamic acid), poly-(1-glutamic acid), poly-(d-aspartic acid), poly-(1-aspartic acid), and copolymers thereof. Polyglutamic acids having molecular weights between about 5,000 to about 100,000 are preferred, with molecular weights between about 20,000 and 80,000 being more preferred and with molecular weights between about 30,000 and 60,000 being most preferred. The polymer is conjugated via an ester linkage to one or more hydroxyls of an epothilone of the invention using a protocol as essentially described by U.S. Patent No. 5,977,163, which is incorporated herein by reference. Preferred conjugation sites include the 3-hydroxyl and the 7-hydroxyl groups.

In still other embodiments, compounds of the invention may be conjugated to a monoclonal antibody. This strategy allows the targeting of the compounds to specific targets. General protocols for the design and use of conjugated antibodies are described in Monoclonal Antibody-Based Therapy of Cancer, Michael L. Grossbard, ed. (1998), which is incorporated herein by reference.

The amount of active ingredient incorporated in the compositions of the invention to produce a single dosage form will vary depending upon the subject treated and the particular mode of administration. The magnitude of the therapeutic dose of the compounds of the invention will vary with the nature and severity of the condition to be treated and with the particular compound and its route of administration. In general, the daily dose range for anticancer activity lies in the range of 0.001 to 100 mg/kg of body weight in a mammal, preferably 0.01 to 80 mg/kg, and most preferably 0.1 to 70 mg/kg, in single or multiple doses. In unusual cases, it may be necessary to administer doses above 100 mg/kg.

In other aspects, there are described methods for treating hyperproliferative diseases, such as cancer, typically, but, not necessarily, in a mammal. In one embodiment, the compounds of the present invention are used to treat cancers of the head and neck which include tumors of the head, neck, nasal cavity, paranasal sinuses, nasopharynx, oral cavity, oropharynx, larynx, hypopharynx, salivary glands, and paragangliomas. The compounds of the present invention are used to treat cancers of the liver and biliary tree, particularly hepatocellular carcinoma. In another embodiment, the compounds of the present invention are used to treat intestinal cancers, particularly colorectal cancer. In another embodiment, the compounds of the present invention are used to treat ovarian cancer. The compounds of the present invention are used to treat small cell and non-small cell lung cancer. In another embodiment, the compounds of the present invention are used to treat breast cancer. The compounds of the present invention are used to treat sarcomas such as fibrosarcoma, malignant fibrous histiocytoma, embryonal rhabdomysocarcoma, leiomysosarcoma, neurofibrosarcoma, osteosarcoma, synovial sarcoma, liposarcoma, and alveolar soft part sarcoma. The compounds of the present invention are used to treat neoplasms of the central nervous systems, particularly brain cancer. The compounds of the present invention are used to treat lymphomas which include Hodgkin's lymphoma, lymphoplasmacytoid lymphoma, follicular lymphoma, mucosa-associated lymphoid tissue lymphoma, mantle cell lymphoma, B-lineage large cell lymphoma, Burkitt's lymphoma, and T-cell anaplastic large cell lymphoma.

The compounds and compositions of the invention may be administered to a subject in need of such treatment a therapeutically effective amount of at least one compound or composition of the invention. Administration to the subject may be repeated as necessary either to contain (i.e. prevent further growth) or to eliminate the cancer. Clinically, practice of the method will result in a reduction in the size or number of the cancerous growths and/or a reduction in associated symptoms (where applicable). Pathologically, practice of the method will produce at least one of the following: inhibition of cancer cell proliferation, reduction in the size of the cancer or tumor, prevention of further metastasis, and inhibition of tumor angiogenesis.

The compounds and compositions of the present invention can be administered in combination therapies, either concurrently with, prior to, or subsequent to one or more other desired therapeutic or medical procedures. The particular combination of therapies and procedures in the combination regimen will take into account compatibility of the therapies and/ or procedures and the desired therapeutic effect to be achieved. Examples of suitable combinations include a compound of the invention and one or more of azacitidine, cladribine, cytarabine, floxuridine, fludarabine phosphate, gemcitabine, pentostatin, uracil mustard, or a nucleoside analog, such as 5 fluorouracil ("5 FU") or a prodrug thereof, such as 5α-deoxy-5 fluoro-N [(pentyloxy)carbonyl]-cytidine (sold commercially under the trade name XELODA^{®} (Roche, Basel Switzerland)) which is an orally administered systemic prodrug of 5'-deoxy-5-fluorouridine (5'-DFUR) which is converted *in vivo* to 5-fluorouracil.

In one embodiment, the compounds and compositions of the present invention are used in combination with another anti-cancer agent or procedure. Illustrative examples of other anti-cancer agents include but are not limited to: (i) alkylating drugs such as mechlorethamine, chlorambucil, cyclophosphamide, melphalan, ifosfamide; (ii) antimetabolites such as methotrexate; (iii) microtubule stabilizing agents such as vinblastin, paclitaxel, docetaxel, and discodermolide; (iv) angiogenesis inhibitors; and (v) cytotoxic antibiotics such as doxorubicon (adriamycin), bleomycin, and mitomycin. Illustrative examples of other anticancer procedures include: (i) surgery; (ii) radiotherapy; and (iii) photodynamic therapy.

In other embodiments, the compounds and/or compositions of the invention may be used in combination with an agent or procedure to mitigate potential side effects from the compounds or compositions, such as diarrhea, nausea and vomiting. Diarrhea may be treated with antidiarrheal agents such as opioids (e.g. codeine, diphenoxylate, difenoxin, and loeramide), bismuth subsalicylate, and octreotide. Nausea and vomiting may be treated with antiemetic agents such as dexamethasone, metoclopramide, diphenyhydramine, lorazepam, ondansetron, prochlorperazine, thiethylperazine, and dronabinol. For those compositions that includes polyethoxylated castor oil such as Cremophor^{®}, pretreatment with corticosteroids such as dexamethasone and methylprednisolone and/or H₁antagonists such as diphenylhydramine HCl and/or H₂ antagonists may be used to mitigate anaphylaxis.

The compounds and/or compositions of the invention may be used to treat non-cancer disorders that are characterized by cellular hyperproliferation. The compounds of the present invention may be used to treat psoriasis, a condition characterized by the cellular hyperproliferation of keratinocytes which builds up on the skin to form elevated, scaly lesions. This method comprises administering a therapeutically effective amount of a compound or composition of the invention to a subject suffering from psoriasis. Administration may be repeated as necessary either to decrease the number or severity of lesions or to eliminate the lesions. Clinically, practice of the method will result in a reduction in the size or number of skin lesions, diminution of cutaneous symptoms (pain, burning and bleeding of the affected skin) and/or a reduction in associated symptoms (e.g., joint redness, heat, swelling, diarrhea abdominal pain). Pathologically, practice of this method will result in at least one of the following: inhibition of keratinocyte proliferation, reduction of skin inflammation (for example, by impacting on: attraction and growth factors, antigen presentation, production of reactive oxygen species and matrix metalloproteinases), and inhibition of dermal angiogenesis.

The compounds of the present invention may be used to treat multiple sclerosis, a condition characterized by progressive demyelination in the brain. Although the exact mechanisms involved in the loss of myelin are not understood, there is an increase in astrocyte proliferation and accumulation in the areas of myelin destruction. At these sites, there is macrophage-like activity and increased protease activity which is at least partially responsible for degradation of the myelin sheath. This method comprises administering a therapeutically effective amount of a compound or composition of the invention to a subject suffering from multiple sclerosis. Such administration may be repeated as necessary to inhibit astrocyte proliferation and/or lessen the severity of the loss of motor function and/or prevent or attenuate chronic progression of the disease. Clinically, practice of this method will result in improvement in visual symptoms (visual loss, diplopia), gait disorders (weakness, axial instability, sensory loss, spasticity, hyperreflexia, loss of dexterity), upper extremity dysfunction (weakness, spasticity, sensory loss), bladder dysfunction (urgency, incontinence, hesitancy, incomplete emptying), depression, emotional lability, and cognitive impairment. Pathologically, practice of the method will result in the reduction of one or more of the following, such as myelin loss, breakdown of the blood-brain barrier, perivascular infiltration of mononuclear cells, immunologic abnormalities, gliotic scar formation and astrocyte proliferation, metalloproteinase production, and impaired conduction velocity.

The compounds and/or compositions of the invention are used to treat rheumatoid arthritis, a multisystem chronic, relapsing, inflammatory disease that sometimes leads to destruction and ankyiosis of affected joints. Rheumatoid arthritis is characterized by a marked thickening of the synovial membrane which forms villous projections that extend into the joint space, multilayering of the synoviocyte lining (synoviocyte proliferation), infiltration of the synovial membrane with white blood cells (macrophages, lymphocytes, plasma cells, and lymphoid follicles; called an "inflammatory synovitis"), and deposition of fibrin with cellular necrosis within the synovium. The tissue formed as a result of this process is called pannus and, eventually the pannus grows to fill the joint space. The pannus develops an extensive network of new blood vessels through the process of angiogenesis that is essential to the evolution of the synovitis. Release of digestive enzymes (matrix metalloproteinases (e.g., collagenase, stromelysin)) and other mediators of the inflammatory process (e.g., hydrogen peroxide, superoxides, lysosomal enzymes, and products of arachidonic acid metabolism) from the cells of the pannus tissue leads to the progressive destruction of the cartilage tissue. The pannus invades the articular cartilage leading to erosions and fragmentation of the cartilage tissue. Eventually there is erosion of the subchondral bone with fibrous ankylosis and ultimately bony ankylosis, of the involved joint. In this aspect of the invention, a therapeutically effective amount of a compound and/or composition of the invention is administered to a subject suffering from rheumatoid arthritis. Such administration may be repeated as necessary to accomplish to inhibit synoviocyte proliferation and/or lessen the severity of the loss of movement of the affected joints and/or prevent or attenuate chronic progression of the disease. Clinically, practice of the present invention will result in one or more of the following: (i) decrease in the severity of symptoms (pain, swelling and tenderness of affected joints; morning stiffness, weakness, fatigue anorexia, weight loss); (ii) decrease in the severity of clinical signs of the disease (thickening of the joint capsule synovial hypertrophy, joint effusion, soft tissue contractures, decreased range of motion, ankylosis and fixed joint deformity); (iii) decrease in the extra-articular manifestations of the disease (rheumatic nodules, vasculitis, pulmonary nodules, interstitial fibrosis, pericarditis, episcleritis, iritis, Felty's syndrome, osteoporosis); (iv) increase in the frequency and duration of disease remission/symptom-free periods; (v) prevention of fixed impairment and disability; and/or (vi) prevention/ attenuation of chronic progression of the disease. Pathologically, practice of the present invention will produce at least one of the following: (i) decrease in the inflammatory response; (ii) disruption of the activity of inflammatory cytokines (such as IL-I, 1NFa, FGF, VEGF); (iii) inhibition of synoviocyte proliferation; (iv) inhibition of matrix metalloproteinase activity, and/or (v) inhibition of angiogenesis.

The compounds and/or compositions of the present invention may be used to threat atherosclerosis and/ or restenosis, particularly in patients whose blockages may be treated with an endovascular stent. Atherosclerosis is a chronic vascular injury in which some of the normal vascular smooth muscle cells ("VSMC") in the artery wall, which ordinarily control vascular tone regulating blood flow, change their nature and develop "cancer-like" behavior. These VSMC become abnormally proliferative, secreting substances (growth factors, tissue-degradation enzymes and other proteins) which enable them to invade and spread into the inner vessel lining, blocking blood flow and making that vessel abnormally susceptible to being completely blocked by local blood clotting. Restenosis, the recurrence of stenosis or artery stricture after corrective procedures, is an accelerated form of atherosclerosis. In this aspect of the invention, a therapeutically effective amount of a compound or composition of the invention may be coated on a stent, and the stent delivered to the diseased artery in a subject suffering from atherosclerosis. Methods for coating a stent with a compound are described for example by U.S. Patent Nos, 6,156,373 and 6,120, 847, which are incorporated herein by reference. Clinically, practice of the present invention will result in one or more of the following: (i) increased arterial blood flow; (ii) decrease in the severity of clinical signs of the disease; (iii) decrease in the rate of restenosis; or (iv) prevention/attenuation of the chronic progression of atherosclerosis. Pathologically, practice of the present invention will produce at least one of the following at the site of stent implantation: (i) decrease in the inflammatory response, (ii) inhibition of V5MC secretion of matrix metalloproteinases; (iii) inhibition of smooth muscle cell accumulation; and (iv) inhibition of V5MC phenotypic dedifferentiation.

Dosage levels that are administered to a subject suffering from cancer or a non-cancer disorder characterized by cellular hyperproliferation are of the order from about I mg/m² to about 200 mg/m², which may be administered as a bolus (in any suitable route of administration) or a continuous infusion (e.g. 1 hour, 3 hours, 6 hours, 24 hours, 48 hours or 72 hours), every week, every two weeks, or every three weeks, as needed. It will be understood, however, that the specific dose level for any particular patient depends on a variety of factors. These factors include the activity of the specific compound employed; the age, body weight, general health, sex, and diet of the subject; the time and route of administration and the rate of excretion of the drug; whether a drug combination is employed in the treatment; and the severity of the condition being treated.

The dosage levels may be from about 10 mg/m² to about 150 mg/m², preferably from about 10 to about 75 mg/m² and more preferably from about 15 mg/m² to about 50 mg/m², once every three weeks as needed and as tolerated. The dosage levels may be from about 1 mg to about 150 mg/m², preferably from about 10 mg/m² to about 75 mg/m² and more preferably from about 25 mg/m² to about 50 mg/m², once every two weeks as needed and as tolerated. The dosage levels may be from about 1 mg/m² to about 100 mg/m², preferably from about 5 mg/m² to about 50 mg/ m² and more preferably from about 10 mg/m² to about 25 mg/m², once every week as needed and as tolerated. The dosage levels may be from about 0.1 to about 25 mg/m², preferably from about 0.5 to about 15 mg/m² and more preferably from about 1 mg/m² to about 10 mg/m², once daily as needed and tolerated.

A detailed description of the invention having been provided above, the following examples are given for the purpose of illustration.

### Comparative Example 1

### Protection of 3,7-diol

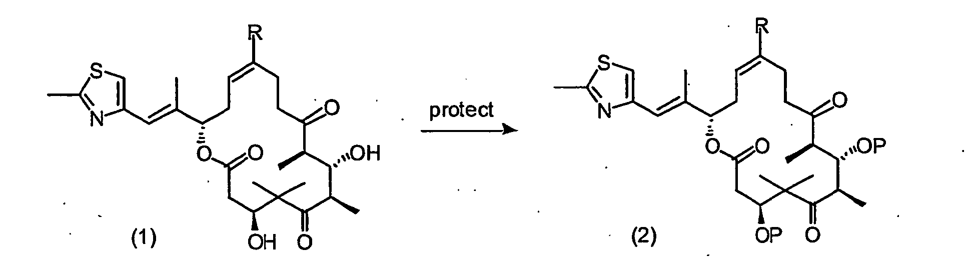

### 3.7-bis-O-(triethylsilyl)-9-oxoepothilone D

### (Compound 2); R = Me; P = Et₃Si)

Chlorotriethylsilane is added to a solution of 9-oxoepothilone D and 2,6-lutidine in dichloromethane at ambient temperature and stirred until the reaction is substantially complete as determined using methods known in the organic synthesis arts (e.g., monitoring by thin-layer chromatography ("TLC") or liquid chromatography ("LC"), typically overnight. The product solution is then diluted with dichloromethane and washed sequentially with water, saturated aqueous sodium bicarbonate solution ("sat. aq. NaHCO₃") and brine, and then dried over magnesium sulfate ("MgSO₄"), filtered, and evaporated to dryness. The desired product is purified by silica gel chromatography using a suitable solvent system, such as, for example, a gradient of ethyl acetate in hexanes.

### Comparative Example 2

### 3,7-bis-O-(tert-butyldimethylsilyl)-9-oxoepothilone D

### (Compound (2); R = Me; P = ^{t}BuMe₂Si)

By analogy to the details provided in Example 1 above, *tert*-Butyldimethylsilyl trifluoromethanesulfonate is added to a solution of 9-oxoepothilone D and 2,6-lutidine in dichloromethane at ambient temperature and stirred overnight. The mixture is diluted with dichloromethane and washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 3

### 3,7-bis-O-(2,2,2-trichloroethoxycarbonyl)-9-oxoepothilone D

### (Compounds (2): R = Me;P = CO₂CH₂CCl₃)

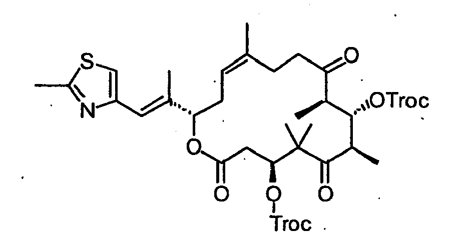

A solution of 9-oxoepothilone D (80 mg) in 1.5 mL of pyridine was treated with 2,2,2-trichloroethyl chloroformate (134 mg) for 16 hours at ambient temperature. The mix was diluted with ethyl acetate, washed successively with sat. NH₄Cl and brine, dried over Na₂SO₄, filtered, and evaporated. Silica gel chromatography (40% ethyl acetate/hexanes) yielded 162 mg of product.

### Comparative Example 4

### 3,7-bis-O-(2,2,2-trichloroethozycarbonyl)-8-epi-9-oxoepothilone D

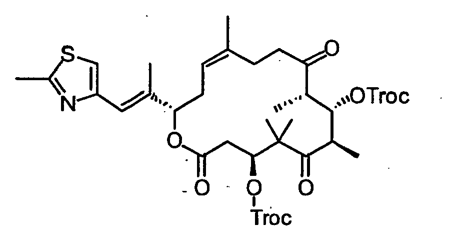

To a solution of 9-oxo-8-*epi*-epothilone D (0.100 g, 0.198 mmol) in pyridine (2.0 ml) was added 2,2,2-trichloroethyl chloroformate (0.27 ml, 0.420 g, 1.980 mmol) followed by dimethylaminopyridine (0.002 g, 0.020 mmol). The resulting solution was stirred at room temperature for 14 hours before partitioning between ethyl acetate (20 ml) and sat. aq. NaHCO₃ (20 ml). The organics were further washed with sat. aq. NH₄Cl (2 x 20 ml) and brine (20 ml) before drying (Na₂SO₄), filtering; and concentrating under reduced pressure. Column chromatography (silica gel, 40% ethyl acetate-hexane) yielded the product as colorless oil (0.125 g, 74%).

### Comparative Example 5

### II. ROUTE 1: KETOREDUCTION AND ELIMINATION

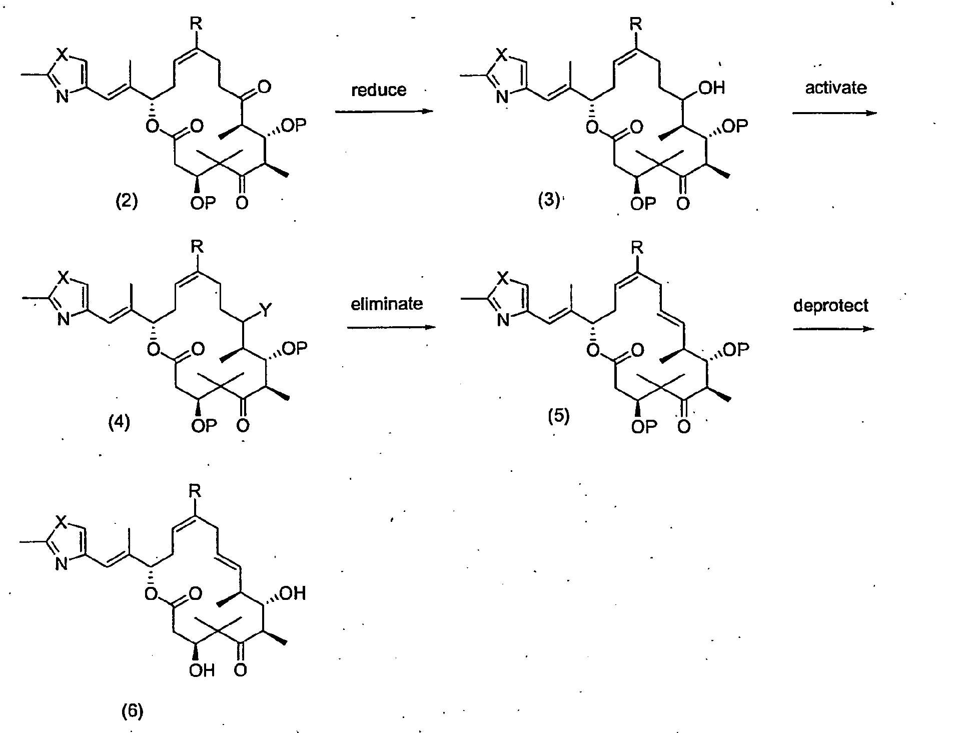

### Step 1. Ketoreduction

### 3,7-bis-O-(triethylsilyl)-9-hydroxyepothilone D

### (Compound (3); R = Me; P = Et₃Si; X = S)

To a solution of 3,7-bis-O-(triethylsilyl)-9-oxoepothilone D in methanol at 0°C is added 1 equivalent of sodium borohydride. The solution is stirred at 0°C for 40 minutes before adding sat. aq. NH₄Cl. The organics are extracted with ethyl acetate and combined before drying (Na₂SO₄) filtering, and concentrating under reduced pressure. Column chromatography (silica, EtOAc-hexane) yields the product.

### Comparative Example 6

### 3,7-bis-O-(tert-butyldimethylsilyl)-9-hydroxyepothilone D

### (Compound (3); R = Me; P = ^{t}BuMe₂Si;X = S)

To a solution of 3,7-bis-O-(*tert*-butyldimethylsilyl)-9-oxoepothilone D in methanol at 0°C is added 1 equivalent of sodium borohydride. The solution is stirred at 0°C for 40 minutes before adding sat. aq. NH₄Cl. The organics are extracted with ethyl acetate and combined before drying (Na₂SO₄), filtering, and concentrating under reduced pressure. Column chromatography (silica, EtOAc-hexane) yields the product.

### Comparative Example 7

### 3,7-bis-O-(2,2,2-trichloroethoxycarbonyl)-9-hydroxyepothilone D

### (Compound (3); R = Me; P = CO₂CH₂CCl₃;X=S)

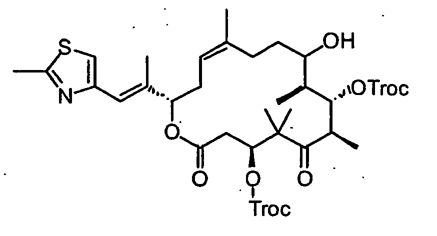

To a solution of 3,7-bis-O-(2,2,2-trichloroethoxycarbonyl)-9-oxoepothilone D in methanol at 0°C is added 1 equivalent of sodium borohydride. The solution is stirred at 0°C for 40 minutes before adding sat. aq. NH₄Cl. The organics are extracted with ethyl acetate and combined before drying (Na₂SO₄), filtering, and concentrating under reduced pressure. Column chromatography (silica, EtOAc-hexane) yields 3,7-bis-O-(2,2,2-trichloroethoxycarbonyl)-9-hydroxyepothilone D.

### Comparative Example 8

### 3,7-bis-O-(2,2,2-trichloroethoxycarbonyl)-8-epi-9-hydroxyepothilone D

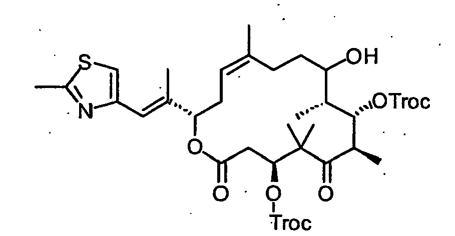

To a solution of 3,7-bis-O-(2,2,2-trichloroethoxycarbonyl)-8-epi-9-oxoepothilone D (0.125 g, 0.131 mmol) in methanol (2.0 ml) at 0°C was added sodium borohydride (0.005 g, 0.131 mmol). The solution was stirred at 0°C for 40 minutes before adding sat. aq. NH₄Cl (15 ml). The organics were extracted with ethyl acetate (3 x 15 ml) and combined before drying (Na₂SO₄), filtering, and concentrating under reduced pressure. Column chromatography (silica, 20% EtOAc-hexane) yielded 3,7-bis-O-(2,2,2-trichloroethoxy-carbonyl)-8-epi-9-hydroxyepothilone D as a colorless oil.

### Comparative Example 9

### Step 2. Activation

### 3,7-bis-O-(triethylsilyl)-9-(methanesulfonyloxy)epothilone D

### (Compound (4); R=Me; P=Et₃Si; X=S; Y=MeSO₂O)

Methanesulfonyl chloride is added to a solution of 3,7-bis-O-(triethylsilyl)-9-hydroxy-epothilone D and 4-(dimethylamino)pyridine in dichloromethane. After stirring for 12 hours, the mixture is diluted with dichloromethane and washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 10

### 3,7-bis-O-(tert-butyldimethylsilyl)-9-(methanesulfonyloxy)epothilone D

### (Compounds (4); R=Me; P=^{t}BuMe₂Si; X=S; Y MeSO₂O)

Methanesulfonyl chloride is added to a solution of 3,7-bis-O-(*tert-*butyldimethylsilyl)-9-hydroxyepothilone D and 4-(dimethylamino)pyridine in dichloromethane. After stirring for 12 hours, the mixture is diluted with dichloromethane and washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 11

### 3,7-bis-O-(2,2,2-trichloroethoxycarbonyl)-9-(methanesulfonyloxy)epothilone D

### (Compound (4); R=Me; P=CO₂CH₂CCl_{2;} X=S; Y=MeSO₂O)

Methanesulfonyl chloride is added to a solution of 3,7-bis-O-(2,2,2-trichloroethoxy-carbonyl)-9-hydroxyepothilone D and 4-(dimethylamino)pyridine in dichloromethane. After stirring for 12 hours, the mixture is diluted with dichloromethane and washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 12

### 3,7-bis-O-(triethylsilyl)-9-(trifluoromethanesulfonyloxy)epothilone D

### (Compound (4); R=Me; P=Et₃Si: X=S; Y=CF₃SO₂O)

A 1.0 M solution of sodium bis(trimethylsilyl)amide in THF is added dropwise to a solution of 3,7-bis-O-(triethylsilyl)-9-hydroxyepothilone D in THF at -78°C. After 30 minutes, trifluoromethanesulfonic anhydride is added dropwise and the mixture is allowed to warm to ambient temperature. The mixture is diluted into ether and washed sequentially with water, sat. aq. NaHCO₃, and brine, then dried over MgSO₄, filtered, and evaporated to dryness. The product is used without further purification.

### Comparative Example 13

### 3,-bis-O-(tert-butyldimethylsilyl)-9-(trifluoromethanesulfonyloxy)epothilone D

### (Compound (4); R=Me; P = ^{t}BuMe₂Si; X=S; Y=CF₃SO₂O)

A 1.0 M solution of sodium bis(trimethylsilyl)amide in THF is added dropwise to a solution of 3,7-bis-O-(*tert*-butyldimethylsilyl)-9-hydroxyepothilone D in THF at -78 °C. After 30 minutes, trifluoromethanesulfonic anhydride is added dropwise and the mixture is allowed to warm to ambient temperature. The mixture is diluted into ether and washed sequentially with water, sat. aq. NaHCO₃, and brine, then dried over MgSO₄, filtered, and evaporated to dryness. The product is used without further purification.

### Comparative Example 14

### 3,7-bis-O-(2,2,2-trichloroethoxycarbonyl)-9-(trifluoromethanesulfonyloxy)epothilone D

### (Compound (4); R=Me: P=CO₂CH₂CCl₃; X=S; Y=CF₃SO₂O)

A 1.0 M solution of sodium bis(trimethylsilyl)amide in THF is added dropwise to a solution of 3,7-bis-O-(2,2,2-trichloroethoxycarbonyl)-9-hydroxyepothilone D in THF at -78 °C. After 30 minutes, trifluoromethanesulfonic anhydride is added dropwise and the mixture is allowed to warm to ambient temperature. The mixture is diluted into ether and washed sequentially with water, sat. aq. NaHCO₃, and brine, then dried over MgSO₄, filtered, and evaporated to dryness. The product is used without further purification.

### Comparative Example 15

### Step 3. Elimination

### 3,7-bis-O-(triethylsilyl)-9,10-dehydroepothilone D via mesylate elimination

### (Compound (5); R=Me; P=Et₃Si; X=S)

A solution of 3,7-bis-O-(triethylsilyl)-9-(methanesulfonyloxy)epothiolone D in THF is cooled to -78 °C and treated with 1 equivalent of a 1.0 M solution of sodium bis(trimethylsilyl)amide in THF. The mixture is allowed to warm to ambient temperature, and is then poured into sat. aq. NH₄Cl and extracted with ethyl acetate. The extract is washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 16

### 3,7-bis-O-(tert-butyldimethylsilyl)-9,10-dehydroepothilone D

### (Compound (5): R = Me, P =^{t}BuMe₂2si; X = S)

A solution of 3,7-bis-O-(*tert*-butyldimethylsilyl)-9-(methanesulfonyloxy)-epothilone D in THF is cooled to -78°C and treated with 1 equivalent of a 1.0 M solution of sodium bis(trimethylsilyl)amide in THF. The mixture is allowed to warm to ambient temperature, and is then poured into sat. aq. NH₄Cl and extracted with ethyl acetate. The extract is washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 17

### 3,7-bis-O-(2,2,2-trichloroethoxycarbonyl)-9,10-dehydroepothilone D

### (Compound (5); R = Me; P = CO₂CH₂CCl₃; X = S)

A solution of 3,7-bis-O-(2,2,2-trichloroethoxycarbonyl)-9-(methanesulfonyloxy)-epothilone D in THF is cooled to -78 °C and treated with 1 equivalent of a 1.0 M solution of sodium bis(trimethylsilyl)amide in THF. The mixture is allowed to warm to ambient temperature, and is then poured into sat. aq. NH₄Cl and extracted with ethyl acetate. The extract is washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 18

### 3.7-bis-O-(triethylsilyl)-9,10-dehydroepothilone D via triflate elimination

### (Compound (5); R = Me; P = Et₃Si; X = S)

A solution of 3,7-bis-O-(triethylsilyl)-9-(trifluoromethanesulfonyloxy)epothilone D in THF is treated with 2,6-di-*tert*-butyl-4-methylpyridine at 0 °C. The mixture is allowed to warm to ambient temperature, then poured into sat. aq. NH₄Cl and extracted with ethyl acetate. The extract is washed sequentially with water, cold 1 N HCl, sat. aq. NaHCO₃, and brine, then dried over MgSO₄, filtered, and evaporated to dryness. The product is purified by silica gel chromatography using a gradient of ethyl acetate in hexanes.

### Comparative Example 19

### Step 4. Deprotection

### 9,10-dehydroepothilone D (via P = Et₃Si)

### (Compound (6); R = Me; X = S)

A solution of 3,7-bis-O-(triethylsilyl)-9,10-dehydroepothilone D in acetonitrile and water is cooled on ice and treated with 48% hydrofluoric acid. The reaction is monitored by thin-layer chromatography. Upon completion, the reaction is neutralized by addition of sat. aq. NaHCO₃ and concentrated to an aqueous slurry. The slurry is extracted with ethyl acetate, and the extract is washed sequentially with water, sat. aq. NaHCO₃, and brine, then dried over MgSO₄, filtered, and evaporated to dryness. The residue is subjected to silica gel chromatography (ethyl acetate-hexanes) to isolate the product.

### Comparative Example 20

### 9,10-dehydroepothilone D (via P ^{t}BuMe₂Si)

### (Compound (6): R = Me; X = S)

A solution of 3,7-bis-O-(*tert*-butyldimethylsilyl)-9,10-dehydroepothilone D in acetonitrile and water is cooled on ice and treated with 48% hydrofluoric acid. The reaction is monitored by thin-layer chromatography. Upon completion, the reaction is neutralized by addition of sat. aq. NaHCO₃ and concentrated to an aqueous slurry. The slurry is extracted with ethyl acetate, and the extract is washed sequentially with water, sat. aq. NaHCO₃, and brine, then dried over MgSO₄, filtered, and evaporated to dryness. The residue is subjected to silica gel chromatography (ethyl acetate-hexanes) to isolate the product.

### Comparative Example 21

### 9,10-dehydroepothilone D (via P = CO₂CH₂CCl₃)

### (Compound (6) ; R = Me; X = S)

Zinc powder is added to a solution of 3,7-bis-O-(2,2,2-trichloroethoxycarbonyl)-9,10-dehydroepothilone D in a mixture of acetic acid and tetrahydrofuran at room temperature. The solution is stirred at room temperature for 1 hour before adding further zinc and stirring at room temperature overnight. The solution is partitioned between ethyl acetate and water and the aqueous phase is extracted with ethyl acetate. The combined organics are washed with brine and dried (Na₂SO₄) before concentrating under reduced pressure. Column chromatography on silica gel using a gradient of ethyl acetate in hexanes yields 9,10-dehydroepothilone D.

### Comparative Example 22

### III. Route 2: Pyrolytic eliminations

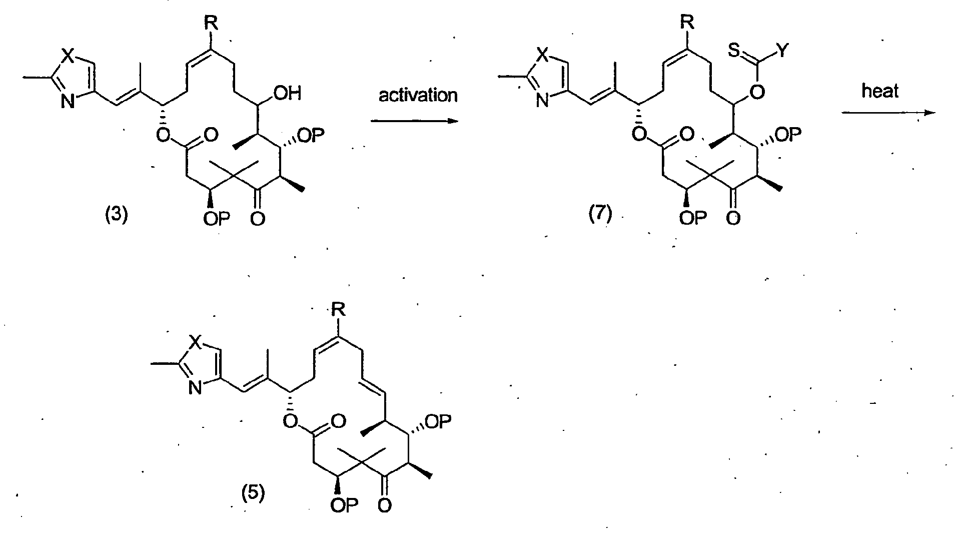

### Step 1. Activation

### 3,7-bis-O-(triethylsilyl)-9-((methylthio)thiocarbonyloxy)epothilone D

### (Compounds (7); R = Me; P = Et₃Si; X = S; Y = S-Me)

A solution of 3,7-bis-O-(triethylsilyl)-9-hydroxyepothilone D in THF is cooled to -78 °C and treated with 1 equivalent of a 1.0 M solution of sodium bis(trimethylsilyl)amide in THF. After 30 minutes, carbon disulfide is added and the mixture is allowed to warm to ambient temperature. After 1 hour, methyl iodide is added and the reaction is allowed to proceed 12 hours, poured into sat. aq. NH₄Cl, and extracted with ethyl acetate. The extract is washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 23

### 3,7-bis-O-(triethylsilyl)-9-((dimethylamino)thiocarbonyloxy)epothilone D

### (Compound (7); R = Me; P = Et₃Si; X = S; Y = NMe₂)

A mixture of 3,7-bis-O-(triethylsilyl)-9-hydroxyepothilone D, dimethylthiocarbamoyl chloride, and pyridine is allowed to react for 12 hours. The mixture is poured into water and extracted with ethyl acetate. The extract is washed sequentially with water, sat. aq. NaHCO₃, and brine, then dried over MgSO₄, filtered, and evaporated to dryness. The product is purified by silica gel chromatography using a gradient of ethyl acetate in hexanes.

### Comparative Example 24

### Step 2. Pyrolysis

### 3,7-bis-O-(triethylsilyl)-9,10-dehydroepothilone D via the xanthate

### (Compound (5); R = Me; P = Et₃Si; X = S)

3,7-bis-O-(triethylsilyl)-9-((methylthio)thiocarbonyloxy)epothilone D is placed under high vacuum (ca. 0.1 torr) and heated at 170 °C under starting material has disappeared as determined by thin-layer chromatographic analysis. The reaction is cooled to ambient temperature, and the residue is subjected to silica gel chromatography (ethyl acetate-hexanes) to isolate the product.

### Comparative Example 25

### 3,7-bis-O-(triethylsilyl)-9,10-dehydroepothilone D via the thiocarbamate

### (Compound (5); R = Me; P = Et₃Si, X = S)

3,7-bis-O-(triethylsilyl)-9-((dimethylamino)thiocarbonyloxy)epothilone D is placed under high vacuum (ca. 0.1 torr) and heated at 170 °C under starting material has disappeared as determined by thin-layer chromatographic analysis. The reaction is cooled to ambient temperature, and the residue is subjected to silica gel chromatography (ethyl acetate-hexanes) to isolate the product.

### Comparative Example 26

### IV. Vinyl triflate reduction

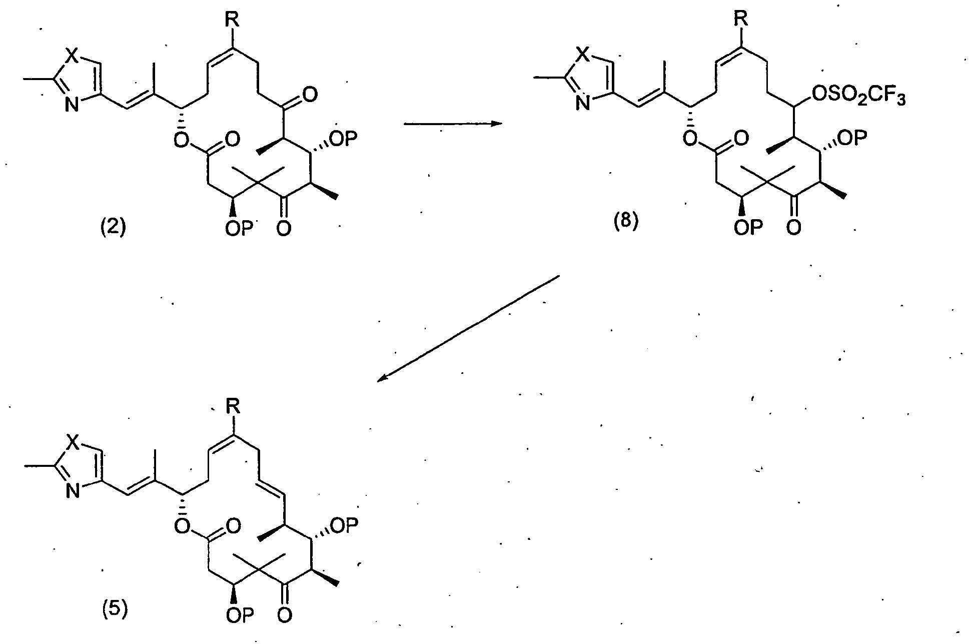

### 3,7-bis-O-(triethylsilyl)-9,10-dehydro-9-(trifluoromethanesulfonyloxy) epothilone D

### (Compound (8); R = Me; P = Et₃Si, X = S)

Trifluoromethanesulfonic anhydride is added dropwise to a solution of 3,7-bis-O-(triethylsilyl)-9-oxoepothilone D and 2,6-di-*tert*-butyl-4-methylpyridine in dichloromethane at 0 °C. After completion of addition, the mixture is allowed to warm slowly to ambient temperature and stirred overnight. The reaction is monitored by thin-layer chromatography, and additional trifluoromethanesulfonic anhydride is added until the starting material has been consumed The mixture is evaporated, and the residue is combined with ethyl ether. The precipitated salts are removed by filtration, and the ethereal solution is washed sequentially with water, ice-cold 1 N HCl, sat. aq. NaHCO₃, and brine, then dried over MgSO₄, filtered, and evaporated to dryness.

### Comparative Example 27

### 3,7-bis-O-(triethylsilyl)-9,10-dehydro-9-(trifluoromethanesulfonyloxy)epothilone D

### (Compound (8); R = Me; P = Et₃Si; X = S)

A solution of 3,7-bis-O-(triethylsilyl)-9-oxoepothilone D in THF is added dropwise to a 1.0 M solution of sodium bis(trimethylsilyl)amide in THF at -78 °C. After completion of addition, the mixture is kept for 30 minutes at -78 °C, and a solution of N-(5-chloro-2-pyridyl)triflimide *(*Organic Syntheses 74: 77-83 (1996)) is added dropwise. The mixture is kept for 2 hours, then allowed to warm slowly to ambient temperature. The mixture is evaporated, and the residue is combined with ethyl ether. The ethereal solution is washed sequentially with water, ice-cold 5% NaOH, and brine, then dried over MgSO₄, filtered, and evaporated to dryness.

### Comparative Example 28

### 3,7-bis-O-(triethylsilyl)-9,10-dehydroepothilone D

### (Compound (5); R = Me; P = Et₃Si; X = S)

A mixture of 3,7-bis-O-(triethylsilyl)-9,10-dehydro-9-(trifluoromethanesulfonyloxy)-epothilone D, tributylamine, palladium acetate, and triphenylphosphine in dimethylformamide is placed under inert atmosphere and degassed by sparging. Formic acid is added via syringe, and the mixture is heated at 60 °C for 1 hour. The mixture is cooled to ambient temperature, diluted into ether, and washed sequentially with water, ice-cold 1 N HCl, sat. aq. NaHCO₃, and brine, then dried over MgSO₄, filtered, and evaporated to dryness. The residue is subjected to silica gel chromatography (ethyl acetate-hexanes) to isolate the product.

### Comparative Example 29

### V. Coupling to the vinyl triflate

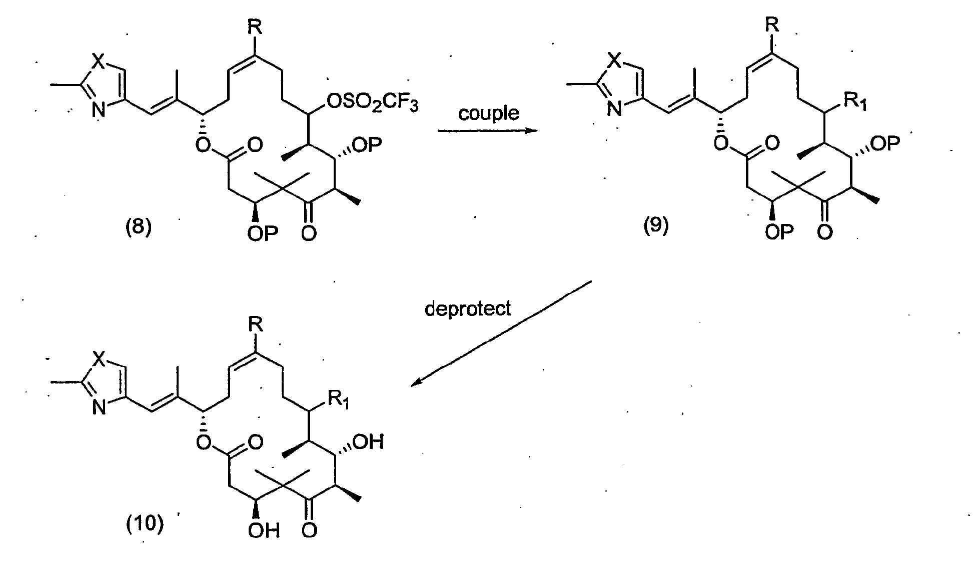

### 3,7-bis-O-(triethylsilyl)-9,10-dehydro-9-allylepothilone D

### (Compound (9); R = Me; R¹ = CH₂CH=CH₂; P = Et₃Si; X = S)

A mixture of tetrakis(triphenylphosphine)palladium(0), and lithium chloride in dry THF is stirred under inert atmosphere for 15 minutes, then a solution of 3,7-bis-O-(triethylsilyl)-9,10-dehydro-9-(trifluoromethanesulfonyloxy)-epothilone D and allyltributyltin in THF is added. The mixture is heated at a gentle reflux for 48 hours. The mixture is cooled to ambient temperature, diluted into ether, and washed sequentially with water, ice-cold 1 N HCl, sat. aq. NaHCO₃, and brine, then dried over MgSO₄, filtered, and evaporated to dryness. The residue is subjected to silica gel chromatography (ethyl acetate-hexanes) to isolate the product.

### Comparative Example 30

### 9,10-dehydro-9-allylepothilone D

### (Compound (10); R = Me; R₁= CH₂CH=CH₂; X=S)

A solution of 3,7-bis-O-(triethylsilyl)-9,10-dehydro-9-allylepothilone D in acetonitrile and water is cooled on ice and treated with 48% hydrofluoric acid. The reaction is monitored by thin-layer chromatography. Upon completion, the reaction is neutralized by addition of sat. aq. NaHCO₃ and concentrated to an aqueous slurry. The slurry is extracted with ethyl acetate, and the extract is washed sequentially with water, sat. aq. NaHCO₃, and brine, then dried over MgSO₄, filtered, and evaporated to dryness. The residue is subjected to silica gel chromatography (ethyl acetate-hexanes) to isolate the product.

### Comparative Example 31

### 9,10-dehydroepothilone D via ring-opened intermediates

### (Compound (10), R = Me; R¹ = H; X = S)

Step 1.Ring opening. A solution of diisobutylaluminum hydride is added to a solution of 3,7-bis-O-(*tert*-butyldimethylsilyl)-9-oxoepothilone D in tetrahydrofuran cooled to -78 °C. The reaction is monitored by TLC or LC, and upon consumption of starting material is quenched by addition of phosphate buffer and allowed to warm to ambient temperature. The mixture is extracted with ethyl acetate, and the extract is dried, filtered, and evaporated. The product is purified by chromatography on silica gel.

Step 2. Protection. A mixture of the product of Step 1, *tert*-butyldimethylsilyl chloride, and imidazole in dimethylformamide is stirred until the reaction is completed as judged by TLC analysis. The mixture is poured into water and extracted with ether. The extract is dried, filtered, and evaporated, and the product is purified by chromatography on silica gel.

Step 3. Vinyl triflate formation. A solution of the product of Step 2 in THF is added dropwise to a solution of sodium bis(trimethylsilyl)amide cooled to -78 °C. After sufficient time for enolate formation, a solution of N-(2-pyridyl)triflimide in THF is added, and the mixture is allowed to warm slowly to ambient temperature. The mixture is poured into water and extracted with ether. The extract is dried, filtered, and evaporated, and the product is used without further purification.

Step 4. Triflate reduction. A mixture of the product of Step 3, tributylamine, palladium acetate, and triphenylphosphine in dimethylformamide is placed under inert atmosphere and degassed by sparging. Formic acid is added via syringe, and the mixture is heated at 60 °C for 1 hour. The mixture is cooled to ambient temperature, diluted into ether, and washed sequentially with water, ice-cold 1 N HCl, sat. aq. NaHCO₃, and brine, then dried over MgSO₄, filtered, and evaporated to dryness. The residue is subjected to silica gel chromatography (ethyl acetate-hexanes) to isolate the product.

Step 5. Removal of the 1-O-TBS group. A solution of the product of Step 4 and' camphorsulfonic acid in methanol and dichloromethane at 0 °C is stirred and monitored by TLC. Upon disappearance of starting material, the reaction is poured into sat. aq. NaHCO₃ and extracted with ethyl acetate. The extract is dried, filtered, and evaporated. The residue is subjected to silica gel chromatography (ethyl acetate-hexanes) to isolate the product.

Step 6. Oxidation. Tetrapropylammonium perruthenate is carefully added to a mixture of the product of Step 5 and N-methylmorpholine oxide in dichloromethane, and the mixture is stirred and monitored by TLC. Upon disappearance of starting material, the reaction is poured into sat. aq. NaHCO₃ and extracted with ethyl acetate. The extract is dried, filtered, and evaporated. The residue is dissolved in a mixture of *tert*-butanol and phosphate buffer and treated with 2-methyl-2-butene and sodium chlorite. The reaction is followed by TLC. Upon disappearance of starting material, the reaction is poured into ethyl acetate, brought to pH 4 by addition of 1 N HCl, and washed sequentially with water and brine. The organic phase is dried, filtered, and evaporated. The residue is subjected to silica gel chromatography (ethyl acetate-hexanes) to isolate the acid product.

Step 7. Removal of the 15-O-TBS group. A solution of tetrabutylammonium fluoride is added to a solution of the product of Step 6 in THF at 0 °C. After completion of the reaction, the mixture is brought to ambient temperature, diluted with ethyl acetate, and washed with water. The organic phase is dried, filtered, and evaporated. The residue is subjected to silica gel chromatography (ethyl acetate-hexanes) to isolate the product.

Step 8. Macrolactonization. Triethylamine and 2,4,6-trichlorobenzoyl chloride are added to a solution of the product of Step 7 in THF at ambient temperature. The mixture is diluted into toluene after 20 minutes and added dropwise to a solution of 4-(dimethylamino)-pyridine in warm toluene over a period of several hours. After completion, the mixture is concentrated, and the product is purified by silica gel chromatography.

Step 9. Deprotection. The product of Step 8 is dissolved in 1:1 trifluoroacetic acid and dichloromethane at 0 °C. The reaction is monitored by TLC, and when complete is concentrated under vacuum. The residue is dissolved in dissolved in ethyl acetate and washed with sat. aq. NaHCO₃, then dried, filtered, and evaporated. The product is purified by silica gel chromatography.

### Comparative Example 32

### 9,10-dehydroepothilone D via ring-opened intermediates, alternate route

### (Compound (10); R = Me; R¹ = H; X = S)

Step 1. Ring opening. A solution of 3,7-bis-O-(*tert*-butyldimethylsilyl)-9-oxo-epothilone D in methanol is treated with 1N NaOH at ambient temperature. The reaction is monitored by TLC or LC, and upon consumption of starting material is quenched by addition of phosphate buffer, pH 4. The methanol is removed by rotary evaporation under vacuum, and the aqueous residue is extracted with ethyl acetate. The extract is dried, filtered, and evaporated. The product is purified by chromatography on silica gel.

Step 2. Esterification. (Trimethylsilyl)diazomethane is added to a solution of the product of Step 1 in ether until a yellow color persists. The solution is concentrated, and the product is purified by chromatography on silica gel.

Step 3. Protection of 15-OH. Chlorotrimethylsilane is added to a solution of the product of Step 2 and trimethylsilylimidazole in dichloromethane at ambient temperature. After 1 hour, the mixture is poured into saturated aqueous NaHCO₃ and extracted with dichloromethane. The extract is dried, filtered, and evaporated. The product is purified by chromatography on silica gel.

Step 4. Vinyl triflate formation. A solution of the product of Step 3 in anhydrous THF is added dropwise to a solution of sodium bis(trimethylsilyl)amide in THF cooled to -78 °C. After sufficient time for enolate formation, a solution of N-(2-pyridyl)triflimide in THF is added, and the mixture is allowed to warm slowly to ambient temperature. The mixture is poured into water and extracted with ether. The extract is dried, filtered, and evaporated. The product is purified by chromatography on silica gel.

Step 5. Triflate reduction. A mixture of the product of step 4, tributylamine, palladium acetate, and triphenylphosphine in dimethylformamide is placed under inert atmosphere and degassed by sparging. Formic acid is added by syringe, and the mixture is heated at 60 °C for 1 hour. The mixture is cooled to ambient temperature, diluted into ether, and washed sequentially with water, ice-cold 1 N HCl, sat. aq. NaHCO₃, and brine. The ether phase is dried, filtered, and evaporated. The product is purified by chromatography on silica gel.

Step 6. Removal of the 15-OTMS group. The product of step 5 is dissolved in a mixture of acetonitrile, water, and acetic acid. The reaction is monitored by TLC or LC, and upon consumption of starting material the mixture is evaporated to dryness under vacuum.

Step 7. Removal of methyl ester. The product of Step 6 is dissolved in methanol and treated with 1 N NaOH at ambient temperature. The reaction is monitored by TLC or LC, and upon consumption of starting material is quenched by addition of phosphate buffer, pH 4. The methanol is removed by rotary evaporation under vacuum, and the aqueous residue is extracted with ethyl acetate. The extract is dried, filtered, and evaporated. The product is purified by chromatography on silica gel.

Step 8. Macrolactonization. Triethylamine and 2,4,6-trichlorobenzoyl chloride are added to a solution of the product of Step 7 in THF at ambient temperature. After 20 minutes, the mixture is diluted into toluene and added dropwise to a solution of 4-(dimethylamino)pyridine in warm toluene over a period of several hours. After completion of addition, the mixture is concentrated. The product is purified by chromatography on silica gel.

Step 9. Deprotection. The product of Step 8 is dissolved in 1:1 trifluoroacetic acid and dichloromethane at 0 °C. The reaction is monitored by TLC or LC, and upon consumption of starting material is evaporated to dryness under vacuum. The residue is dissolved in ethyl acetate and washed with sat. aq. NaHCO₃ followed by brine. The ethyl acetate phase is dried, filtered, and evaporated. The product is purified by chromatography on silica gel.

### Comparative Example 33

### 9,10-dehydroepothilone H₂ via ring-opened intermediates

### (Compound 10); R = Me; R¹ H; X = O)

Step 1. Protection. A solution of 9-oxoepothilone H₂ and 2,6-lutidine in dichloromethane is treated with *tert*-butyldimethylsilyl trifluoromethanesulfonate at ambient temperature. After stirring overnight, the mixture is diluted with dichloromethane and washed sequentially with water, sat. aq. NaHCO₃, and brine. The solution is dried, filtered, and evaporated, and the product is purified by silica gel chromatography.

Step 2. Ring opining. A solution of 3,7-bis-O-(*tert*-butyldimethylsilyl)-9-oxo-epothilone H₂ in methanol is treated with 1 N NaOH at ambient temperature. The reaction is monitored by TLC or LC, and upon consumption of starting material is quenched by addition of phosphate buffer, pH 4. The methanol is removed by rotary evaporation under vacuum, and the aqueous residue is extracted with ethyl acetate. The extract is dried, filtered, and evaporated. The product is purified by chromatography on silica gel.

Step 3. Esterification. (Trimethylsilyl)diazomethane is added to a solution of the product of Step 2 in ether until a yellow color persists. The solution is concentrated, and the product is purified by chromatography on silica gel.

Step 4. Protection of 15-OH. Chlorotrimethylsilane is added to a solution of the product of Step 3 and trimethylsilylimidazole in dichloromethane at ambient temperature. After 1 hour, the mixture is poured into saturated aqueous NaHCO₃ and extracted with dichloromethane. The extract is dried, filtered, and evaporated. The product is purified by chromatography on silica gel.

Step 5. Vinyl triflate formation. A solution of the product of Step 4 in anhydrous THF is added dropwise to a solution of sodium bis(trimethylsilyl)amide in THF cooled to -78 °C. After sufficient time for enolate formation, a solution of N-(2-pyridyl)triflimide in THF is added, and the mixture is allowed to warm slowly to ambient temperature. The mixture is poured into water and extracted with ether. The extract is dried, filtered, and evaporated. The product is purified by chromatography on silica gel.

Step 6. Triflate reduction. A mixture of the product of step 5, tributylamine, palladium acetate, and triphenylphosphine in dimethylformamide is placed under inert atmosphere and degassed by sparging. Formic acid is added by syringe, and the mixture is heated at 60 °C for 1 hour. The mixture is cooled to ambient temperature, diluted into ether, and washed sequentially with water, ice-cold 1 N HCl, sat. aq. NaHCO₃, and brine. The ether phase is dried, filtered, and evaporated. The product is purified by chromatography on silica gel.

Step 7. Removal of the 15-OTMS group. The product of step 6 is dissolved in a mixture of acetonitrile, water, and acetic acid. The reaction is monitored by TLC or LC, and upon consumption of starting material the mixture is evaporated to dryness under vacuum.

Step 8. Removal of methyl ester. The product of Step 7 is dissolved in methanol and treated with 1 N NaOH at ambient temperature. The reaction is monitored by TLC or LC, and upon consumption of starting material is quenched by addition of phosphate buffer, pH 4. The methanol is removed by rotary evaporation under vacuum, and the aqueous residue is extracted with ethyl acetate. The extract is dried, filtered, and evaporated. The product is purified by chromatography on silica gel.

Step 9. Macrolactonization. Triethylamine and 2,4,6-trichlorobenzoyl chloride are added to a solution of the product of Step 8 in THF at ambient temperature. After 20 minutes, the mixture is diluted into toluene and added dropwise to a solution of 4-(dimethylamino)pyridine in warm toluene over a period of several hours. After completion of addition, the mixture is concentrated. The product is purified by chromatography on silica gel.

Step 10. Deprotection. The product of Step 9 is dissolved in 1:1 trifluoroacetic acid and dichloromethane at 0 °C. The reaction is monitored by TLC or LC, and upon consumption of starting material is evaporated to dryness under vacuum. The residue is dissolved in ethyl acetate and washed with sat. aq. NaHCO₃ followed by brine. The ethyl acetate phase is dried, filtered, and evaporated. The product is purified by chromatography on silica gel.

### Comparative Example 34

### (Compound (24); R = Me; X = S)

Step 1. Ketone reduction. A solution of the product of Example 32, Step 3 in methanol is cooled to 0 °C and treated with 1 equivalent of sodium borohydride. The solution is stirred at 0 °C for 40 minutes before adding sat. aq. NH₄Cl. The organics are extracted with ethyl acetate and the extract is dried, filtered, and concentrated to dryness. The product is purified by silica gel chromatography.

Step 2. Xanthate formation. A solution of the product of Step 1 in THF is cooled to -78 °C and treated witch 1 equivalent of a 1.0 M solution of sodium bis(trimethylsilyl)amide in THF. After 30 minutes, carbon disulfide is added and the mix is allowed to warm to ambient temperature. After 1 hour, methyl iodide is added and the reaction is allowed to proceed for 12 hours, poured into sat. aq. NH₄Cl, and extracted with ethyl acetate. The extract is washed sequentially with water, sat. aq. NaHCO₃, and brine, then dried, filtered, and evaporated. The product is purified by silica gel chromatography.

Step 3. Pyrolytic elimination. The product from Step 2 is placed under high vacuum (ca. 0.1 torr) and heated at 150-200°C until starting material has been consumed as determined by TLC analysis. The reaction is cooled to ambient temperature, and the residue is subjected to silica gel chromatography to isolate the product.

### Comparative Example 35

### (Compound (24); R = Me; X = O)

Step 1. Ketone reduction. A solution of the product of Example 33, Step 4 in methanol is cooled to 0 °C and treated with 1 equivalent of sodium borohydride. The solution is stirred at 0 °C for 40 minutes before adding sat. aq. NH₄Cl. The organics are extracted with ethyl acetate and the extract is dried, filtered, and concentrated to dryness. The product is purified by silica gel chromatography.

Step 2. Xanthate formation. A solution of the product of Step 1 in THF is cooled to -78°C and treated with 1 equivalent of a 1.0 M solution of sodium bis(trimethylsilyl)amide in THF. After 30 minutes, carbon disulfide is added and the mix is allowed to warm to ambient temperature. After 1 hour, methyl iodide is added and the reaction is allowed to proceed for 12 hours, poured into sat. aq. NH₄Cl, and extracted with ethyl acetate. The extract is washed sequentially with water, sat. aq. NaHCO₃, and brine, then dried, filtered, and evaporated. The product is purified by silica gel chromatography.

Step 3. Pyrolytic elimination. The product from Step 2 is placed under high vacuum (ca. 0.1 torr) and heated at 150-200°C until starting material has been consumed as determined by TLC analysis. The reaction is cooled to ambient temperature, and the residue is subjected to silica gel chromatography to isolate the product.

### Comparative Example 36

### 2-(pivalolyloxymethyl)-4-(chloromethyl)thiazole

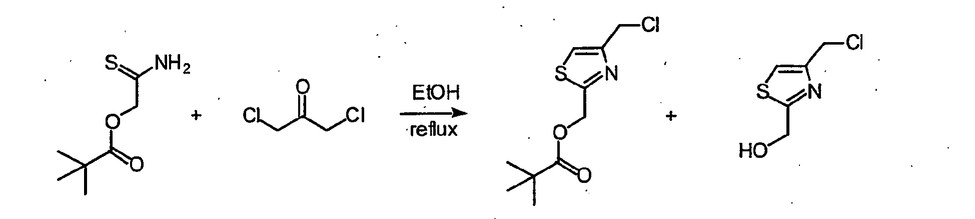

A mixture of 30 mmol of 1,3-dichloroacetone, and 30 mmol of 2-(pivaloyloxy)-thioacetamide was dissolved into 21 ml of absolute alcohol, and refluxed overnight under nitrogen. The resulted mixture was concentrated under vacuum, and the residue was dissolved into 100 ml of water. The resulted aqueous solution was neutralized to pH = 8 before it was extracted with ether (200 ml X 3). The combined organic layers were washed sequentially with saturated NaHCO₃, water, and brine. The organic phase was dried with MgSO₄, filtered, and evaporated to yield a brown syrup. Chromatography on silica gel (gradient from 2% to 10% acetone in hexane) yielded 200 mg of 2-(pivalolyloxymethyl)-4-chloromethylthiazole, and 800 mg of 2-(hydroxymethyl)-4-(chloromethyl)thiazole.

### Comparative Example 37

### 2-(tert-butyldimethylsilyloxymethyl)-4-(chloromethyl)thiazole

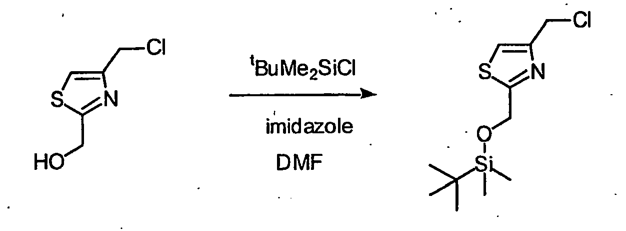

A mixture of 800 mg of (2-hydroxymethyl)-(4-chloromethyl)thiazole (4.89 mmol) and 1.33 g of imidazole (18.56 mmol) was dissolved into 10 ml of DMF. The resulting solution was cooled to 0 °C before 1.47 g of *t*-butyldimethylsilyl chloride (9.78 mmol) was added in portionwise. The resulting solution was allowed to warm to ambient temperature, and the reaction was continued for another 4 h before it was quenched by adding 30 ml of sat. aq. NaHCO₃ solution. The resulting mixture was extracted with 50 ml X 2 of ethyl acetate. The combined organic extracts were washed sequentially with water 20 ml X 3, and brine. The organic phase was dried with MgSO₄, filtered, and evaporated to yield a brown syrup. Chromatography on silica gel (gradient from 1% to 10% acetone in hexane) yielded. 800 mg of 2-(*tert*-butyldimethylsilyloxymethyl)-4-(chloromethyl)-thiazole.

### Comparative Example 38

### 2-(tert-butyldimethylsilylomethyl)-4-thiazolyl) methyl]tributylphosphonium chloride

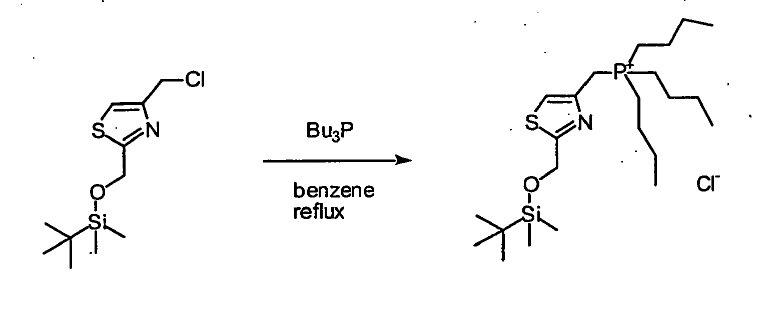

To a solution of 557.0 mg of 2-(*tert*-butyldimethylsilyloxymethyl)-4-(chloromethyl)thiazole (2.0 mmol) in 3 ml of benzene was added tri-n-butyl phosphine dropwise under nitrogen atmosphere. The resulting solution was refluxed under nitrogen overnight. The solution was then concentrated under vacuum, and the residue was crystallized by adding a mixture of 1:1 (v/v) of ether and hexane. The solid was then filtered, washed with a small amount of hexane before it was dried over vacuum to provided 800 mg of the phosphonium salt as a white solid.

### Comparative Example 39

### Preparation of 21-hydroxy-9,10-dehydro-epothilone D

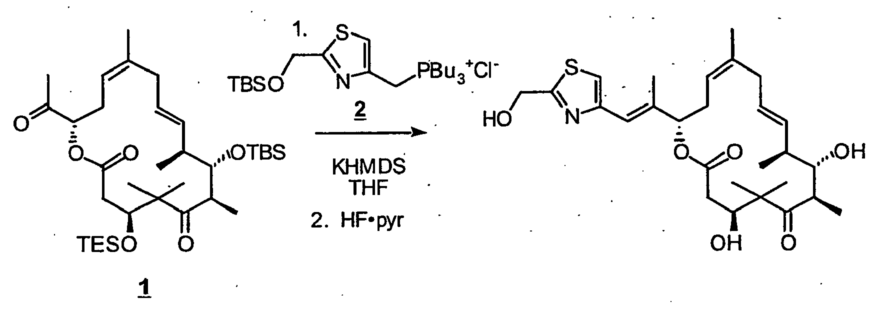

A 0.5 M solution of potassium bis(trimethylsilyl)amide in toluene (2.4 mL) was added to a solution of (2-(*tert*-butyldimethylsilyloxy)methylthiazol-4-yl)methyltri-n-butyl-phosphonium chloride (0.58 g) in 1 mL of tetrahydrofuran (THF) at -20 °C. The solution was allowed to warm to 0 °C over 30 minutes, then cooled to -78 °C and a solution of the ketone 1 (128 mg) (A. Rivkin et al., J. Am. Chem. Soc. 2003 125: 2899-2901) was added. The mixture was allowed to warm to -40 °C over 1 hour, at which time the reaction was judged complete by thin-layer chromatographic analysis. The reaction was quenched by addition of sat. aq. Ammonium chloride and extracted with ethyl acetate. The extract was washed with brine, dried over magnesium sulfate, filtered, and evaporated. The residue was chromatographed on silica gel (20:1 hexanes/ether) to provide 150 mg of pure protected product.

The protected product (150 mg) was dissolved in 3 mL of THF and treated with hydrogen fluoride-pyridine (2 mL) at 0 °C. The reaction was allowed to warm to ambient temperature over 1 hour, and is then kept for 4 hours. Methoxytrimethylsilane (15 mL) was added slowly, and the mixture was stirred overnight. The mixture was evaporated to dryness, and the residue was chromatographed on silica gel (3:1 to 1:1 hexanes/acetone) to provide 64 mg of pure 21-hydroxy-9,10-dehydroepothilone D.

### Comparative Example 40

### Preparation of 21-azido-9,10-dehydro-epothilone D

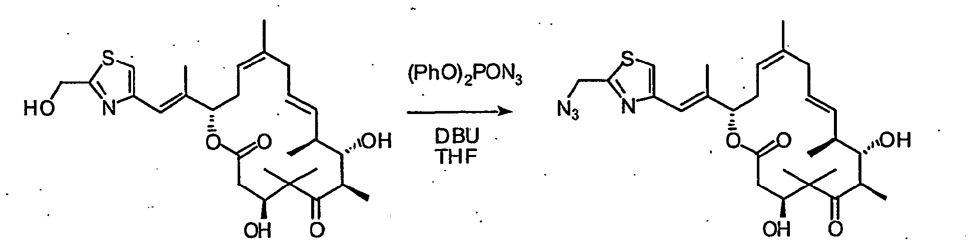

A solution of 21-hydroxy-9,10-dehydroepothilone D (30 mg) in 1.5 mL of dry THF was cooled to 0 °C and treated with diphenyl phosphorylazide (15.3 µL) over 1 minute. After 5 minutes, 1,8-diaza[5.4.0]bicycloundec-7-ene (DBU) (8.8 µL) was added, and the reaction was stirred at 0 °C for 2 hours, then warmed to ambient temperature. After 18 hours, thin-layer chromatographic analysis indicated remaining 21-hydroxy-9,10-dehydroepothilone D, and so the mixture was cooled to 0 °C and treated with addition diphenyl phosphorylazide (2 µL). The mixture was stirred an additional 30 minutes at 0 °C and then at ambient temperature for 1.5 hours. The solution was poured into 30 mL of ethyl acetate and washed with 2x 10 mL of water. The combined aqueous washes were, extracted with ethyl acetate (2x 15 mL), and the extracts were combined, dried over magnesium sulfate, filtered, and evaporated. The crude material was chromatographed on silica gel to provide 9 mg of product.

### Comparative Example 41

### Preparation of 21-amino-9,10-dehydro-epothilone D

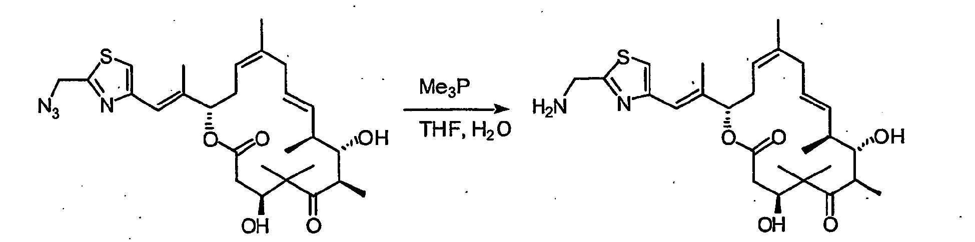

A solution of 21-azido-9,10-dehydroepothilone D (14 mg) and trimethylphosphine (33 µL of a 1M solution in THF) in 0.3 mL of THF was stirred for 5 minutes, then treated with 80 µL of water and stirred an additional 3 hours. The mixture was evaporated to dryness, and the residue was chromatographed on silica gel (10% methanol in chloroform) to yield 8 mg of 21-amino-9,10-dehydroepothilone D. Exact mass: calc. For C₂₇H₄₁N₂O₅S (M+H) = 505.2731, obs. = 505.2719. ¹³C-NMR (CDCl₃, 100 MHz): δ 218.6, 172.8, 170.5, 152.4, 138.1, 137.3, 131.1, 129.8, 120.3, 119.4, 116.1, 78.2, 75.4, 71.5, 53.2, 44.6, 43.9, 40.0, 39.5, 34.8, 31.8, 23.5, 22.4, 19.2, 17.6, 15.8, 15.0.

### Comparative Example 42

### Preparation of 21- hydroxy-9,10-dehydro-26-trifluoroepothilone D

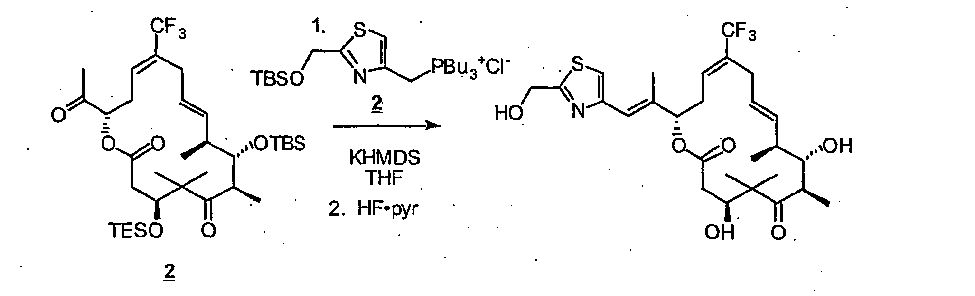

A 0.5 M solution of potassium bis(trimethylsilyl)amide in toluene (1.5 mL) was added dropwise over 10 minutes to a solution of (2-(*tert*-butyldimethylsilyloxy)-methylthiazol-4-yl)methyltri-n-butyl-phosphoium chloride (0.641 g) in 3 mL of tetrahydrofuran (THF) at -30°C. The solution was allowed to warm to 0 °C over 40 minutes, then cooled to -70°C and a solution of the ketone 3 (85 mg) (A. Rivkin et al., J. Am. Chem. Soc. 2003 125: 2899-2901) in 1 mL of THF was added dropwise over 10 minutes. After 20 minutes, the mixture was allowed to warm to -30 °C over 1 hour. The reaction was quenched by addition of sat. aq. ammonium chloride and extracted with ethyl acetate. The extract was washed with brine, dried over sodium sulfate, filtered, and evaporated. The residue was chromatographed on silica gel, eluting sequentially with 0, 2, 4, 6, and 8% ether in heptanes, to provide 67 mg of pure protected product.

The protected product (67 mg) was dissolved in 1.5 mL of THF and treated with hydrogen fluoride-pyridine (0.6 mL) at 0 °C. After 20 minutes, the reaction was allowed to warm to ambient temperature, kept for 3.5 hours, then cool back to 0 °C. Methoxytrimethylsilane (6 mL) was added slowly, and the mixture was brought to ambient temperature and evaporated to an oil. The oil was chromatographed on silica gel (0, 10, 20, 30, 40, 50, 60, 70, 80, and 90% ethyl acetate in hexanes) to provide pure 21-hydroxy-9,10-dehydro-26-trifluoroepothilone D. LC/MS: *m*/*z* 560 [M+H].

### Comparative Example 43

### Preparation of 21-azido-9,10-dehydro-26-trifluoroepothilone D

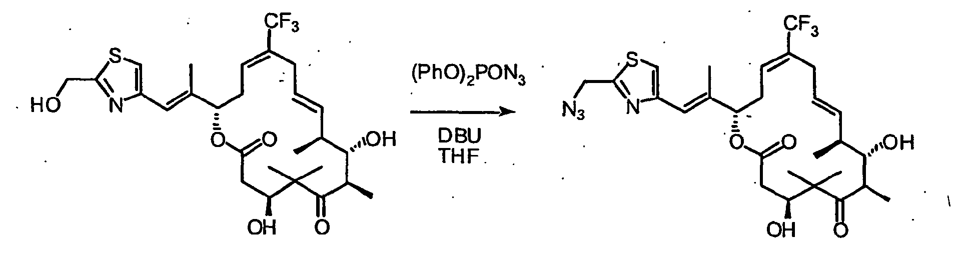

A solution of 21-hydroxy-9,10-dehydro-26-trifluoroepothilone D (20 mg) in 0.5 mL of dry THF was cooled to 0 °C and treated with diphenyl phosphorylazide (10 µL). After 5 minutes, 1,8-diaza[5.4.0]bicycloundec-7-ene (DBU) (5 µL) was added, and the reaction was warmed to ambient temperature and stirred over night. The reaction mixture was applied directly to a column of silica gel, which was then eluted with 0, 10, 20, 30, 40, 50, 60, 70 and 80% ethyl acetate in hexanes to provide the product. MS: calc. for C₂₇H₃₈N₄O₅S [M+H] = 530.2563; obs. 585.2797.

### Comparative Example 44

### Preparation of 21-amino-9,10-dehydro-26-trifluoroepothilone D

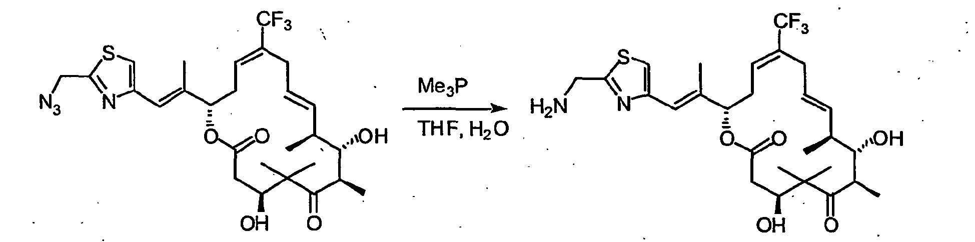

A solution of 21-azido-9,10-dehydro-26-trifluoroepothilone D (13 mg) was cooled to 0 °C and treated with trimethylphosphine (28 µL of a 1 M solution in THF) in 0.5 mL of THF was stirred for 5 minutes, then treated with 100 µL of water and allowed to warm to ambient temperature over 1.5 hours. The reaction mixture was applied directly to a column of silica gel, which was then eluted with 0, 2, 4, 6, 8, and 10% methanol in dichloromethane containing 1% triethylamine to provide impure product. A second chromatography on silica gel, eluting with 0, 25, 50, 75, and 100% ethyl acetate in hexanes followed by 0, 2, and 5% methanol in dichloromethane containing 1% triethylamine provided pure product. LC/MS: *m*/*z* 559 [M+H].

### Comparative Example 45

### 17-des(2-methyl-4-thiazolyl)-17-(2-pyridyl)-9,10-dehydropeothilone D (KOSN 1632)

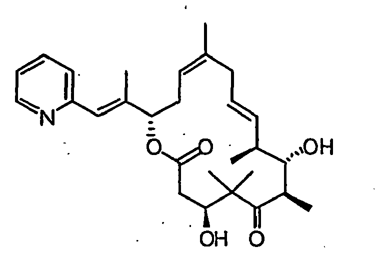

(2-Pyridyl)methyltri-n-butylphosphonium chloride was prepared as follows. To a solution of 10 mmol of 2-(chloromethyl)pyridine in 15 ml of benzene was added 10 mmol of tri-n-butylphosphine dropwise under nitrogen. The resulting solution was refluxed for 18 hour before it was cooled down to ambient temperature. The solution was then concentrated under vacuum; all the necessary measures have been taken to reduce any contact with air. A white solid was formed by adding diethyl ether to the residue. The mother liquor was filtered away, and the white solid was washed several times with diethyl ether under nitrogen, the solid was then dried under vacuum to give a white powder as the final product.

17-des(2-methyl-4-thiazolyl)-17-(2-pyridyl)-9,10-dehydropeothilone D was prepared according to the method of Example 42, replacing (2-(*tert*-butyldimethyl-silyloxy)-methylthiazol-4-yl)methyltri-n-butyl-phosphonium chloride with (2-pyridyl)methyltri-n-butylphosphonium chloride, and allowing the reaction to warm to -10 °C prior to quenching. ¹³C-NMR (100 MHz, CDCl₃): δ 218.6, 170.6, 155.9, 149.0, 141.1, 137.4, 136.3, 131.1, 129.8, 125.2, 124.0, 121.4, 120.4, 77.9, 75.3, 71.4, 53.5, 44.5, 40.0, 39.6, 34.8, 31.9, 23.6, 22.7, 18.6, 17.3, 15.7, 14.8.

### Comparative Example 46

### 17-des(2-methyl-4-thiazolyl)-17-(2-quinolyl)-9,10-dehydropeothilone D

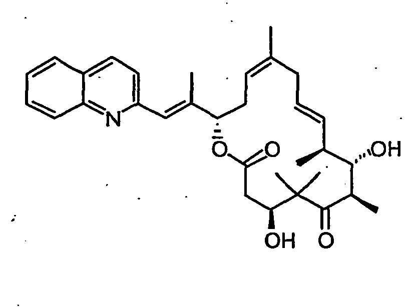

Prepared according to the method of Example 42, replacing (2-(*tert-*butyldimethyl-silyloxy)-methylthiazol-4-yl)methyltri-n-butyl-phosphonium chloride with (2-quinolyl)methyltri-n-butylphosphonium chloride, and allowing the reaction to warm to ambient temperature and kept for 3 hrs prior to quenching. ¹³C-NMR (100 MHz, CDCl₃): δ 218.5, 170.7, 156.2, 147.7, 142.9, 137.5, 136.2, 131.2, 129.9, 129.8, 128.7, 127.5, 126.6, 126.3, 125.5, 122.2, 120.5, 78.0, 75.2, 71.5, 53.6, 44.4, 40.0, 39.6, 34.9, 32.0, 23.6, 23.1, 18.5, 17.2, 16.1, 14.6.

### Comparative Example 47

### 17-des(2-methyl-4-thiazolyl)-17-(2-benzothiazolyl)-9,10-dehydropeothilone D (KOSN 1635)

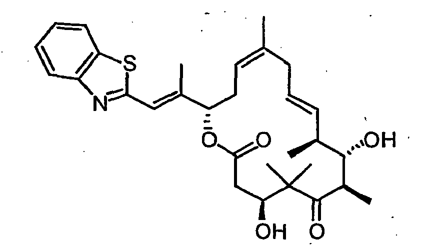

Prepared according to the method of Example 42, replacing (2-(*tert-*butyldimethyl-silyloxy)-methylthiazol-4-yl)methyltri-n-butyl-phosphonium chloride with (2-benzothiazolyl)methyltri-n-butylphosphonium chloride, and allowing the reaction to warm to ambient temperature and kept for 2 days prior to quenching. ¹³C-NMR (100 MHz, CDCl₃): δ 218.6, 170.4, 164.6, 152.7, 145.4, 137.9, 134.9, 131.1, 129.7, 126.4, 125.2, 122.8, 121.4, 119.8, 119.5, 77.6, 75.6, 71.8, 53.2, 44.8, 40.1, 39.4, 34.8, 31.6, 23.5; 22.6, 19.3, 17.6, 16.9, 15.1.

### Example 48

### Cytotoxicity data

### Cell lines and culture conditions

Human breast carcinoma cell line MCF-7, multi-drug resistant breast carcinoma cell line NCI/ADR, were obtained from the National Cancer Institute. Human non-small cell lung cancer cell line A549 and human ovarian cancer cell line SKOV-3 were obtained from American Type Culture Collection (Manassas, VA). All cell lines were maintained in RPMI-1640 medium (Gibco/BRL, Rockville, MD) supplemented with 2 mM L-glutamine, 25 mM HEPES and 10% FBS (Hyclone, Logan, UT). Cells were maintained in a humidified incubator at 37°C in 5% CO₂.

### Cytotoxicity assays

Tumor cells were seeded in 100 µl at 5000 (MCF-7), 7500 (NCI/ADR), 5000 (A549) and 7500 (SKOV3) cells per well in 96-well plates. Cells were allowed to adhere for 24 hours. Each compound ranging from 0.001 to 1000 nM in 100 µl was added to cells in duplicate wells. After 3 days, cells were fixed at 4°C for 1 hour with 10% trichloroacetic acid and then stained with 0.2% sulforhodamine B (SRB)/1% acetic acid for 20 minutes at room temperature. The unbound dye was rinsed away with 1% acetic acid, and the bounded SRB was then extracted with 200 µl of 10 mM Tris base. The absorbance was measured at 515 nm using a 96-well microtiter plate reader (Spectra Max 250, Molecular Devices). The IC₅₀ values were calculated using a KaleidaGraph program. The experiments were performed twice. The results are shown in the following Table 1.

**Table 1 (comparative example)**

| Analog | MCF-7 | NCI/ADR | A549 | SKOV3 |
|---|---|---|---|---|
| Epothilone D | 6.8 | 23 | 25 | 39 |
| 9,10-dehydroepothilone D | 2 | 6.2 | 4.7 | 6.2 |
| 21-OH 9,10-dehydroepothilone D | 2.3 | 14 | 3.8 | 5.7 |
| 21-NH₂ 9,10-dehydroepothilone D | 3.2 | 25 | 5.9 | 5.7 |
| 26-trifluoro 9,10-dehydroepothilone D | 5.3 | 33 | 11 | 22 |
| 21-OH 26-trifluoro 9,10-dehydroepothilone D | 3.6 | 65 | 6.1 | 7.9 |
| 21-NH₂ 26-trifluoro 9,10-dehydro epothilone D | 9.7 | 330 | 35 | 40 |
| 17-des(2-methyl-4-thiazolyl)-17-(2-pyridyl)-9,10-dehydroepothilone D | 0.87 | 4.6 | 3.3 | 4.4 |
| 17-des(2-methyl-4-thiazolyl)-17-(2-quinolyl)-9,10-dehydroepothilone D | 8.9 | 24 | 25 | 19 |
| 17-des(2-methyl-4-thiazolyl)-17-(2-benzothiazolyl)-9,10-dehydroepothilone D | 1.1 | 5.6 | 4.2 | 3.4 |

### Example 49

### Additional 9,10-dehydroepothilone D Analogs

Following the procedure of Examples 39 (when R = methyl) and 42 (when R = CF₃), the additional compounds shown in Table 2 were made by replacing (2-(tert-butyldimethyl-silyloxy)-methylthiazol-4-yl)methyltri-n-butyl-phosphonium chloride with the corresponding phosphonium chloride. Cytotoxicity data (Table 2) was obtained as described in Example 48. Cytochrome P450 inhibition data (Table 3) was obtained using the initial rate method against BFC substrate.

(4-Methoxypyridin-2-yl)methyltributylphosphonium chloride was prepared as follows. To a solution of 770 mg (4.9 mmol) of 2-(chloromethyl)-4-methoxypyridine in 7 ml of benzene was added 1.22 ml of tri-n-butylphosphine (4.9 mmol) dropwise under nitrogen. The resulting solution was refluxed for 18 hour before it was cooled down to ambient temperature. The solution was then concentrated under vacuum; all the necessary measures have been taken to reduce any contact with air. A slightly yellow solid was formed by adding diethyl ether to the residue. The mother liquor was filtered away, and the yellow solid was washed several times with diethyl ether under nitrogen, the solid was then dried under vacuum to give the phosphonium salt as 1.2 g of yellow powder. (5-Methyl-isoxazol-3-yl)methyltributyl phosphonium chloride was prepared as follows: 3-(chloromethyl)-5-methyl isoxazole (0.30 g, 2.28 mmol, 1 eq.) was dissolved in 6 mL benzene in a 100 mL oven-dried RBF. Tributylphosphine (0.57 mL, 2.28 mmol, 1 eq.) was added via syringe, and the solution was refluxed for 15 hours under a nitrogen atmosphere. The solution was cooled to room temperature and the volume reduced *in vacuo.* Et₂O was added to precipitate out the desired phosphonium salt. The ether supernatant was decanted off, and the white solid dried under vacuum overnight (0.760 g, 99% yield).

¹³C-NMR data for selected compounds shown in Table 2 are as follows:
**KOSN 1703** ¹³C NMR (CDCl₃, 100 MHz) δ 218.6, 170.5, 154.7, 147.3, 141.0, 137.1, 135.5, 134.4, 130.7, 129.8, 124.2, 123.7, 120.5, 77.7, 75.4, 70.8, 61.8, 53.7, 44.6, 39.7, 39.6, 34.8, 31.8, 23.5, 22.8, 18.1, 17.4, 15.9, 15.0.
**KOSN 1727** ¹³C NMR (CDCl₃, 100 MHz) δ 218.6, 170.5, 168.8, 152.3, 137.4, 131.2, 129.8, 120.3, 119.5, 117.1, 78.2, 75.5, 71.7, 67.0, 59.9, 53.7, 53.1, 44.6, 40.1, 39.4, 34.8, 31.9, 23.5, 22.4, 19.4, 17.5, 15.9, 15.0.
**KOSN 1756** ¹³C NMR (CDCl₃, 100 MHz) δ 218.6, 170.6, 165.9, 157.2, 150.1, 141.2, 137.3, 131.1, 129.8, 125.1, 120.5, 110.2, 107.5, 77.7, 75.2, 71.2, 55.1, 53.6, 44.4, 39.9, 39.6, 34.8, 31:9, 23.5, 22.9, 18.3, 17.3, 15.8, 14.7
**KOSN 1674** ¹³C NMR (CDCl₃, 100 MHz) δ 218.3, 170.3, 144.6, 137.8, 135.9, 132.9, 131.0, 129.8, 127.8, 124.3, 119.8, 119.4, 118.8, 110.0, 75.9, 75.4, 71.6, 53.4, 44.7, 39.9, 39.5, 34.9, 31.6, 23.6, 22.7. 18.6, 17.3, 15.0, 14.5.
**KOSN 1724:** ¹³C NMR (CDCl₃, 100 MHz) δ 218.6, 170.3, 168.9, 159.9, 142.9, 137.6, 131.1, 129.6, 119.9, 113.6, 102.1, 77.7, 75.5, 71.7, 53.0, 44.7, 40.0, 39.4, 34.8, 31.6, 23.4, 22.2, 19.4, 17.5, 16.1, 15.0, 12.1.
**KOSN 1673** ¹³C NMR (CDCl₃, 100 MHz) δ 218.2, 170.0, 155.5, 149.0, 139.4, 136.4, 132.1, 130.2, 127.6, 125.6, 124.1, 121.6, 76.2, 75.0, 70.8, 53.8, 44.7, 39.5, 39.1, 30.8, 28.4, 22.6, 17.6, 17.4, 15.7, 14.8.

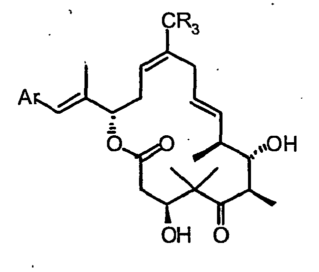

**Table 2**

| | | | IC₅₀ (nM) | | | |
|---|---|---|---|---|---|---|
| Compound ID | Ar | R | MCF-7 | NCI/ ADR | A549 | Skov |
| KOSN-1727 | 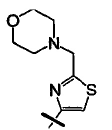 | H | 390 | 410 | 420 | 490 |
| KOSN-1712 | 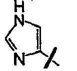 | H | 20 | 220 | 31 | 37 |
| KOSN-1759 | 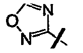 | H | 36 | 61 | 42 | 51 |
| KOSN-1724 | 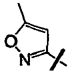 | H | 2.5 | 6.1 | 5.3 | 5.3 |
| KOSN-1674 | 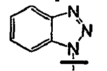 | H | 34 | 180 | 42 | 50 |
| KOSN-1690 | 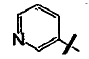 | H | 35 | 110 | 38 | 39 |
| KOSN-1697 | 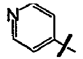 | H | 57 | 320 | 350 | 300 |
| KOSN-1703 | 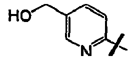 | H | 0.61 | 7.1 | 2.4 | 4.5 |
| KOSN-1765 | 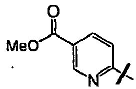 | H | 44 | 360 | 350 | 220 |
| KOSN-1756 | 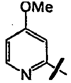 | H | 2.1 | 4.9 | 4 | 4 |
| KOSN-1673 | 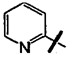 | F | 4.3 | 19 | 15 | 9.6 |

### EXAMPLE 50

### Pharmacological Parameters

Cytochrome P450 inhibition measurements were performed using a commercially-available kit (BD GenTest, Woburn, MA), using BFC as substrate against CYP3A4. The IC₅₀ values in micromolar so obtained are listed in Table 3.

**Table 3**

| | IC₅₀ (nM) | | |
|---|---|---|---|
| Compound | CYP3A4 | CYP2C9 | CYP2C19 |
| Epothilone D | 2.4 | 9 -12 | 1.6 - 3.4 |
| *Trans*-9,10-dehydroepothilone D | 1.6 | 10.8 | 2 |
| 21-OH-26-F₃-*trans*-9,10-dehydroepothilone D | 1 | 17.3 | 20.5 |
| 21-NH₂- *trans*-9,10-dehydroepothilone D | 0.61 | 0.83 | 0.63 |
| 26-F₃- *trans*-9,10-dehydroepothilone D | 2 | 14.4 | 3.2 |
| 21-OH- *trans*-9,10-dehydroepothilone D | 0.8 | 22 | 5.6 |
| KOSN 1635 | 3.12 | 4.1 | 3.27 |
| KOSN 1632 | 2.52 | 15 | 3.05 |
| KOSN 1673 | 3.5 | 9 | 4.7 |
| KOSN 1703 | 6 | 4 | 10.2 |
| KOSN 1756 | 4.8 | 7.1 | 1.9 |
| KOSN 1724 | 7.1 | 12.9 | 10.9 |

Compound solubility was determined as follows: 5 µL of stock solution (at 20 mg/mL in DMSO) was added to 95 µL of phosphate-buffered saline. After being vortexed for about 10 seconds, the solution/suspension was filtered through a 0.45 micron filter and the amount of analyte was quantitated by HPLC. Solubilities (mg/mL) determined were as follows: epothilone D, <0.06; *trans*-9,10-dehydroepothilone D, <0.06; 21-OH-26-F₃- *trans*-9,10-dehydroepothilone D, 0.1; 21-amino- *trans*-9,10-dehydroepothilone D, >0.6; 21-amino-26-F₃-*trans*-9,10-dehydroepothilone D, >0.6; 26-F₃- *trans*-9,10-dehydroepothilone D, <0.06; 21-OH- *trans*-9,10-dehydroepothilone D, 0.1; KOSN 1635, <0.02; KOSN 1632, 0.12.

Half-lives of compounds in fresh mouse and human plasma were determined by dissolving the compounds in DMSO and adding aliquots to samples of mouse or human plasma. At Time Zero, aliquotted 75 µl from each sample and added 150 µl of acetonitrile. Centrifuged the white precipitate at 13500 rpm for 3 minutes. Transferred all the clear supernatant into another microcentrifuge tube and repeated the centrifuging step (13500rpm/3 min). Carefully pipetted 150 µl of the clear supernatant into labelled HPLC sample tubes and tried to avoid pipetting any protein pellets. The samples were analyzed by HPLC to measure remaining compound at each time point by comparison with authentic standards. The half-lives (minutes) in mouse and human plasma, respectively, were determined to be: epothilone D: 49, >1440; 21-OH-26-F₃-*trans*-9,10-dehydroepothilone D: 379, >1440; 21-amino- *trans*-9,10-dehydroepothilone D: 59, >1440; 26-F₃- *trans*-9,10-dehydroepothilone D: 163, >1440; 21-OH- *trans*-9,10-dehydroepothilone D: 1059, >1440; KOSN 1635: 58, >1440; KOSN 1632: 28,>1440. Using frozen mouse plasma, *trans*-9,10-dehydroepothilone D had a half-life of 47 minutes. In fresh human plasma, *trans*-9,10-dehydroepothilone D had a half life > 1440 min.

Plasma protein binding studies were performed as follows. Compounds were dissolved in DMSO, then diluted into either fresh or thawed samples of mouse or human plasma and incubated at 37 °C. Time points of 50 µL were mixed with 100 µl of acetonitrile and centrifuged at ~14000 rpm for 3 minutes. A 100 µL aliquot was removed from the supernatant and set aside for analysis to indicate compound in the total fraction. The remaining supernatant was transferred onto a Microcon ultrafiltration device fitted with a YM10 membrane and centrifuged to separate protein-bound material. A 50 µL aliquot of the protein-free filtrate was mixed with 100 µL of acetonitrile, centrifuged at ~14000 rpm for 3 minutes, then 100 µL of the supernatant was collected and analyzed to determine the amount of compound not protein bound. The amount of compound in the total sample and in the protein-free fraction were determined by HPLC analysis against authentic standards.

These plasma protein binding studies indicated the following percentages of compound protein bound: epothilone D: 98.5%; *trans*-9,10-dehydroepothilone D, 96.6%; 26-trifluoro-*trans*-9,10-dehydroepothilone D, 96.5%; 21-amino-*trans*-9,10-dehydro-epothilone D, 89.5%; 21-OH-26-F₃-*trans*-9,10-dehydroepothilone D, 92.8%; 21-OH-*trans*-9,10-dehydroepothilone D, 94.2%; KOSN 1635, 99.7%; KOSN 1632, 89.4%.

While the preferred embodiment of the invention has been illustrated and described, it will be appreciated that various changes can be made therein.

## Claims

1. A compound of the following formula (I) or a pharmaceutically acceptable salt thereof: 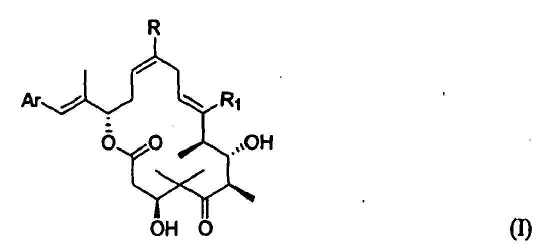 wherein R is H, C₁-C₄ loweralkyl or C₁-C₄ loweralkyl substituted with one or more groups independently selected from halo, hydroxyl, amino and azido;
R₁ is H, or is substituted or unsubstituted C₁-C₄ loweralkyl, C₂-C₄ loweralkenyl, or C₂-C₄ loweralkynyl, which may be substituted with one or more groups independently selected from halo, hydroxyl, amino and azido; and
Ar is 3-isoxazolyl, which may be unsubtituted, monosubstituted or disubustituted with groups independently selected from hydroxy, hydroxyC₁-C₄alkyl, halo, cyano, oxo, alkylimino, amino, alkylamino, dialkylamino, acylaminoalkyl, C₁-C₄ alkoxy, thioalkoxy, C₁-C₄ loweralkyl, cycloalkyl or C₁-C₄ haloalkyl.

2. A compound of Claim 1 wherein R is unsubstituted C₁-C₄ loweralkyl or C₁-C₄ loweralkyl substituted with one or more groups independently selected from halo, hydroxyl, amino and azido.

3. A compound of Claim 1 wherein R is methyl or trifluoromethyl.

4. A compound of Claim 1 wherein R₁ is H, C₁-C₄ loweralkyl, or C₁-C₄ loweralkyl substituted with one or more groups independently selected from halo, hydroxyl, amino and azido.

5. A compound of Claim 3 wherein Ar is 5-methyl-3-isoxazoyl.

6. A compound as claimed in Claim 1 of the formula:

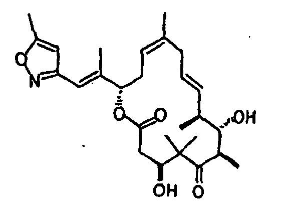

7. A compound as claimed in Claim 1 of the formula:

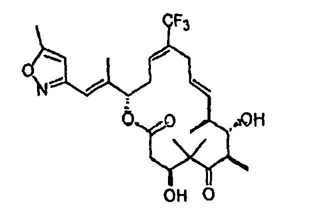

8. A composition comprising an amount of a compound of Claim 1 effective to reduce cellular hyperproliferation in a human or animal subject when administered thereto, together with a pharmaceutically acceptable carrier.

9. A composition of Claim 8 which comprises one or more active agents in addition to the compound of Claim 1.

10. A compound of any of Claims I to 7 for use as a pharmaceutical.

11. The use of a compound of any of Claims 1 to 7 in the manufacture of a medicament for the treatment of hyperproliferative disease.

12. The use of Claim 11 wherein the hyperproliferative disease is selected from cancer, psoriasis, multiple sclerosis, rheumatoid arthritis, atherosclerosis and restenosis.

13. The use of Claim 11 wherein the hyperproliferative disease is a cancer selected from the group consisting of breast cancer, colorectal cancer, and non-small cell lung cancer.

## Patentansprüche

1. Verbindung der folgenden Formel (I) oder ein pharmazeutisch verträgliches Salz davon: 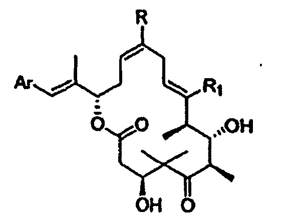 worin R H, C₁-C₄-Niederalkyl oder C₁-C₄-Niederalkyl ist, substituiert mit einer oder mehreren Gruppe(n), die unabhängig aus Halogen, Hydroxyl, Amino und Azido ausgewählt ist/sind;
R₁ H ist oder substituiertes oder nicht substituiertes C₁-C₄-Niederalkyl, C₂-C₄-Niederalkenyl oder C₂-C₄-Niederalkynyl ist, das mit einer oder mehreren Gruppe(n) substituiert sein kann, die unabhängig aus Halogen, Hydroxyl, Amino und Azido ausgewählt ist/sind; und
Ar 3-Isoxazolyl ist, welches mit Gruppen nicht substituiert, monosubstituiert oder disubustituiert sein kann, die unabhängig ausgewählt sind aus: Hydroxy, Hydroxy-C₁-C₄-alkyl, Halogen, Cyano, Oxo, Alkylimino, Amino, Alkylamino, Dialkylamino, Acylaminoalkyl, C₁-C₄-Alkoxy, Thioalkoxy, C₁-C₄-Niederalkyl, Cycloalkyl oder C₁-C₄-Halogenalkyl.

2. Verbindung nach Anspruch 1, worin R nicht substituiertes C₁-C₄-Niederalkyl oder C₁-C₄-Niederalkyl, substituiert mit einer oder mehreren Gruppe(n) ist, die unabhängig aus Halogen, Hydroxyl, Amino und Azido ausgewählt ist/sind.

3. Verbindung nach Anspruch 1, worin R Methyl oder Trifluormethyl ist.

4. Verbindung nach Anspruch 1, worin R₁ H, C₁-C₄-Niederalkyl oder C₁-C₄-Niederalkyl, substituiert mit einer oder mehreren Gruppe(n), die unabhängig aus Halogen, Hydroxyl, Amino und Azido ausgewählt ist/sind, ist.

5. Verbindung nach Anspruch 3, worin Ar 5-Methyl-3-isoxazoyl ist.

6. Verbindung nach Anspruch 1 der Formel:

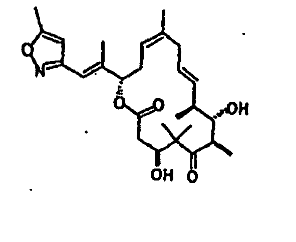

7. Verbindung nach Anspruch 1 der Formel:

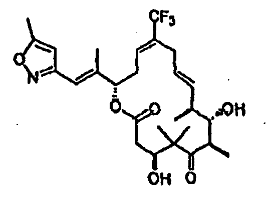

8. Zusammensetzung, umfassend eine Menge einer Verbindung nach Anspruch 1, die zur Reduktion einer zellulären Hyperproliferation in einem Menschen oder Tier wirksam ist, wenn sie zusammen mit
einem pharmazeutisch verträglichen Träger an ihn/es verabreicht wird.

9. Zusammensetzung nach Anspruch 8, welche einen oder mehr Wirkstoff(e) zusätzlich zur Verbindung nach Anspruch 1 umfasst.

10. Verbindung nach einem der Ansprüche 1 bis 7 zur Anwendung als ein Pharmazeutikum.

11. Verwendung einer Verbindung nach einem der Ansprüche 1 bis 7 in der Herstellung eines Arzneimittels zur Behandlung einer hyperproliferativen Erkrankung.

12. Verwendung nach Anspruch 11, worin die hyperproliferative Erkrankung aus Krebs, Psoriasis, multipler Sklerose, rheumatoider Arthritis, Atherosklerose und Restenose ausgewählt ist.

13. Verwendung nach Anspruch 11, worin die hyperproliferative Erkrankung eine Krebserkrankung ist, die aus der Gruppe ausgewählt ist, die aus Brustkrebs, kolorektalem Krebs und nicht kleinzelligem Lungenkrebs besteht.

## Revendications

1. Composé de la formule (I) suivante ou un sel pharmaceutiquement acceptable du même: 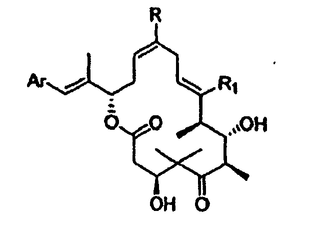 où R est H, alkyle inférieur C₁-C₄ ou alkyle inférieur C₁-C₄ substitué avec un ou plusieurs groupes sélectionnés indépendamment parmi halo, hydroxyle, amino et azido;
R₁ est H ou alkyle inférieur C₁-C₄ substitué ou non substitué, alcényle inférieur C₂-C₄ ou alcynyle inférieur C₂-C₄ qui peut être substitué avec un ou plusieurs groupes sélectionnés indépendamment parmi halo, hydroxyle, amino et azido; et
Ar est 3-isoxazolyle qui peut être non substitué, monosubstitué ou di-substitué avec des groupes sélectionnés indépendamment parmi hydroxy, hydroxy-alkyle C₁-C₄, halo, cyano, oxo, alkylimino, amino, alkylamino, dialkylamino, acylaminoalkyle, alcoxy C₁-C₄, thioalcoxy, alkyle inférieur C₁-C₄, cycloalkyle ou haloalkyle C₁-C₄.

2. Composé selon la revendication 1, dans lequel R est un alkyle inférieur C₁-C₄ non substitué ou un alkyle inférieur C₁-C₄ substitué avec un ou plusieurs groupes sélectionnés indépendamment parmi halo, hydroxyle, amino et azido.

3. Composé de la revendication 1, dans lequel R est méthyle ou trifluorométhyle.

4. Composé de la revendication 1, dans lequel R₁ est H, alkyle inférieur C₁-C₄ ou alkyle inférieur C₁-C₄ substitué avec un ou plusieurs groupes sélectionnés indépendamment parmi halo, hydroxyle, amino et azido.

5. Composé de la revendication 3, dans lequel Ar est 5-méthyl-3-isoxazolyle.

6. Composé tel que revendiqué dans la revendication 1, de la formule:

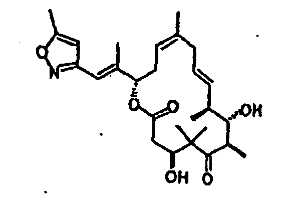

7. Composé tel que revendiqué dans la revendication 1, de la formule:

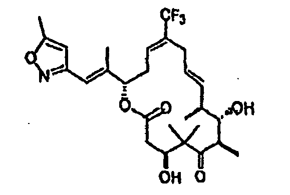

8. Composition comprenant une quantité d'un composé de la revendication 1, efficace pour réduire une hyperprolifération cellulaire chez un sujet humain ou animal lorsque administrée à celui-ci, avec un excipient pharmaceutiquement acceptable.

9. Composition de la revendication 8, qui comprend un ou plusieurs agents actifs en plus du composé de la revendication 1.

10. Composé de l'une quelconque des revendications 1 à 7 à utiliser comme un produit pharmaceutique.

11. L'utilisation d'un composé de l'une quelconque des revendications 1 à 7 dans la fabrication d'un médicament pour le traitement d'une maladie hyperproliférative.

12. L'utilisation de la revendication 11, dans laquelle la maladie hyperproliférative est sélectionnée parmi cancer, psoriasis, sclérose en plaques, polyarthrite rhumatoïde, athérosclérose et resténose.

13. L'utilisation de la revendication 11, dans laquelle la maladie hyperproliférative est un cancer sélectionné parmi le groupe consistant en cancer du sein, cancer colorectal et cancer du poumon non à petites cellules.
